# EUROPEAN PATENT APPLICATION

(11) **EP 4 119 660 A1**
(43) Date of publication of application: **18.01.2023**
(21) Application number: 21186231.3
(22) Date of filing: 16.07.2021
(51) Int. Cl.: C12N 9/16, A23K 10/16

(54) **PHYTASE VARIANTS**

(71) Applicant: AB Enzymes Oy, 05200 Rajamäki (FI)
(72) Inventor: JUNTUNEN, Kari, 05200 Rajamäki (FI); AHOLA, Pihla, 05200 Rajamäki (FI); BENSON, Sven, 70569 Stuttgart (DE); LORENZ, Lenz, 70569 Stuttgart (DE); METZGER, Tara, 64293 Darmstadt (DE); KÜHN, Imke, 64293 Darmstadt (DE); PURANEN, Terhi, 05200 Rajamäki (FI); PALOHEIMO, Marja, 05200 Rajamäki (FI)
(74) Representative: Espatent Oy

(57) **Abstract**

A thermostable phytase variant, a method for its manufacturing, an animal feed, a feed supplement, a dry and a liquid formulation, a method of degrading phytic acid, a method of manufacturing a phytase variant, a method of manufacturing a feed pellet, and a recombinant host cell configured to produce at least one polypeptide are disclosed, wherein the phytase variant comprises amino acid substitutions and has at least 80% amino acid sequence identity with amino acids of SEQ ID NO: 1.

## Description

### TECHNICAL FIELD

The present disclosure generally relates to field of enzyme technology. The disclosure relates particularly, though not exclusively, to phytase variants having an improved thermostability. The present phytase variants are useful in various applications where degradation of phytate is desired, such as in feedstuffs.

### BACKGROUND

This section illustrates useful background information without admission of any technique described herein representative of the state of the art.

Phytic acid (phytate, inositol hexakisphosphate, IP6) is found in many plants where it functions as storage of phosphate. Phosphate stored in IP6 molecules can be released as inorganic phosphate. When inorganic acid is released from myo-inositol hexakisphosphate (IP6) it is converted first to myo-inositol pentakisphosphate (IP5) and further via myo-inositol tetrakisphosphate (IP4), myo-inositol trisphosphate (IP3), myo-inositol bisphosphate (IP2) to myo-inositol monophosphate (IP1).

Phytases are a group of phosphatase enzymes that catalyse the hydrolysis of phytic acid. Commercially available phytases belong to the histidine acid phosphatase (HAP) protein family. The phytases belonging to the HAP protein family share conserved N-terminal active site hepta-peptide motif RHGXRXP and the catalytically active HD-dipeptide at the C-terminus. Histidine acid phosphatases are part of a larger superfamily of histidine phosphatases. They share a conserved catalytic core centred on a histidine, which becomes phosphorylated during the reaction. PFAM motif His_Phos_2 (PF00328) represents branch 2 of the histidine phosphatase superfamily, the branch containing mainly acid phosphatases and phytases.

Phytases are used in feeds to improve phosphate availability from feed ingredients of plant origin (e.g. wheat, barley, corn, soybean) by phytate degradation. This is in particular of interest for monogastric animals like poultry, fish and pigs, because intestinal phytate degradation in the upper intestinal tract of these animals is limited. This limitation not only restricts utilisation of phosphorus, but also the availability of other nutrients due to the chelating effect of inositol phosphates.

It is an object of the present disclosure to provide phytase variants that exhibit phytase activity and that have thermostability that allows their use in industrial processes. Another object of the is to provide phytase variants with improved properties when used in industrial processes. Yet another object of the present disclosure is to provide phytase variants that can be used in enzyme compositions for phytate degradation.

### SUMMARY

The appended independent claims define the scope of protection. Any example or technical description of a product and/or method in the description and/or drawings not covered by the claims is presented herein not as an embodiment of the invention, but as background art or as an example which is useful for understanding the claimed invention.

According to a first aspect is provided a variant polypeptide of an E. *coli* phytase comprising an amino acid sequence having at least 80% amino acid sequence identity with SEQ ID NO: 1, wherein the variant has:
a glutamic acid at the position 224;
phytase activity; and
wherein the amino acid numbering corresponds to the amino acid numbering of the SEQ ID NO: 1.

Advantageously, the present variant polypeptide (also called herein a phytase variant) has improved thermostability compared to the parent phytase having the SEQ ID NO: 1. Improved thermostability of the present phytase variant means that the variant remains functionally stable (for example retains its enzyme activity fully or partially) and/or chemically stable (for example does not unfold or denature) after being exposed to high temperatures as compared to the parent phytase. The improved thermostability can be used to an advantage when high level of recovery of the phytase is needed after demanding process steps such as exposures to a high temperature where phytase activity may be lost in those variants that do not share the structural properties of the present phytase variant. Further, the improved thermostability is advantageous when a manufacturing process of a product containing the present phytase variant comprises further steps in which high temperature is involved, such as in extrusion. As shown in the examples provided below, the claimed phytase variant has a thermostability that is improved compared to the parent phytase enzyme, allowing the present variant to withstand demanding processing conditions and/or stability against proteases.

Advantageously, a substitution of the position 224 with E can be used to enhance the strength of electrostatic interactions in the structure. Preferably, a substitution of the position 224 with E can be used to enhance the strength of a salt bridge between the backbone hydrogen of G233 and the side chain of the residue 224. The second negatively charged sidechain oxygen of E224 could result in a more beneficial electrostatic interaction with the hydrogen 'HE1' of the amino acid W40 compared to the hydrogens of Q at that position.

In an embodiment the variant polypeptide has less than 100% amino acid sequence identity with SEQ ID NO: 1.

In an embodiment the present phytase variant has at least IP6 degrading activity.

In an embodiment is provided a functional fragment of the present phytase variant, the functional fragment having phytase activity.

In an embodiment the variant polypeptide comprises at least one further amino acid substitution at a position selected from 30, 31, 35, 56, 67, 74, 80, 94, 107, 118, 120, 126, 140, 154, 174, 176, 177, 179, 180, 182, 183, 200, 202, 204, 211, 212, 227, 253, 271, 276, 277, 285, 287, 302, 315, 344, 352, 380, and 395. The substitution may be made to the variant polypeptide artificially, such as by genetic engineering.

In an embodiment the E. *coli* phytase is a 6-phytase, or a variant of such a 6-phytase. According to this embodiment the amino acid sequence of the present variant polypeptide is derived from an E. *coli* 6-phytase or its variant.The amino acid in position 224 may naturally be glutamic acid, or it is substituted to a glutamic acid.

In an embodiment the variant polypeptide has an increased thermostability, and/or an increased IP6 degrading activity, compared to a polypeptide having the amino acid sequence SEQ ID NO: 1. Thermostability and IP6 degrading activity can be analysed as described in the Examples.

In an embodiment the variant polypeptide has a relative activity of at least 1 compared to the SEQ ID NO: 1.

In another embodiment the variant polypeptide has a relative activity of at least 1.03, 1.05, 1.1, 1.15, 1.2, 1.25, 1.3, 1.35, 1.4, 1.45, 1.5, 2, 2.5, 3, or 3.5 compared to the SEQ ID NO: 1. In another embodiment the variant polypeptide has a relative activity of at least 1.15 compared to the SEQ ID NO: 1. In another embodiment the variant polypeptide has a relative activity of at least 1.4 compared to the SEQ ID NO: 1. In another embodiment the variant polypeptide has a relative activity of at least 1.8 compared to the SEQ ID NO: 1.

In an embodiment the at least one further amino acid substitution results into the presence of at least one of the following amino acids: 30R/K, 31C, 35N, 56S, 67I/T, 74Q, 80P, 94L, 107N, 118L, 120R, 126H, 140C, 154N/E, 174E, 176P, 177C, 179K, 180N, 182K, 183A, 200C/I, 202S, 204N, 211V, 212G/A, 227E, 253Y/Q, 2711, 276M, 277A, 285E, 287S, 302A, 315G, 344D/E, 352M, 380P, and 395A, wherein the amino acid numbering corresponds to the amino acid numbering of the SEQ ID NO: 1. The variant polypeptide may thus be modified such that it contains at least one amino acid as specified above. In an embodiment the at least one further amino acid substitution is a substitution selected from Q30R/K, D31C, D35N, A56S, V67I/T, K74Q, S80P, R94L, D107N, T118L, S120R, N126H, V140C, D154N/E, Q174E, N176P, L177C, L179K, K180N, E182K, K183A, V200C/I, A202S, D204N, W211V, S212G/A, Q227E, V253Y/Q, L271I, T277A, K276M, Q285E, Q287S, G302A, E315G, N344D/E, L352M, A380P, and G395A.

In an embodiment the variant polypeptide comprises at least one additional amino acid substitution selected from 75C/V, 114T, 137E, 141T, 142D, 146R, 157G, 204C, 211W, 253Q, 267R, and 341P.

In an embodiment the variant polypeptide polypeptide comprises:
a. the amino acids 30R, 74Q, 94L, 118L, 176P, 179K, 180N, 204N, 211V, 212G, 224E, 253Y, 315G, and 380P; or
b. the amino acids 30R, 94L, 118L, 176P, 179K, 180N, 204N, 211V, 212G, 224E, 253Y, 315G, 352M, and 380P; or
c. the amino acids 30R, 74Q, 94L, 118L, 176P, 179K, 180N, 204N, 211V, 212G, 224E, 253Y, 315G, 352M, and 380P; or
d. the amino acids 30R, 74Q, 80P, 94L, 118L, 176P, 179K, 180N, 204N, 211V, 212G, 224E, 227E, 253Y, 276M, 287S, 315G, 352M, and 380P; or
e. the amino acids 30R, 35N, 80P, 94L, 118L, 176P, 179K, 180N, 204N, 211V, 212G, 224E, 227E, 253Y, 276M, 287S, 315G, 352M, and 380P; or
f. the amino acids 30R, 80P, 94L, 118L, 176P, 179K, 180N, 183A, 204N, 211V, 212G, 224E, 227E, 253Y, 276M, 287S, 315G, 352M, and 380P; or
g. the amino acids 30R, 80P, 94L, 118L, 176P, 179K, 180N, 204N, 211V, 212G, 224E, 227E, 253Y, 276M, 287S, 315G, 352M, and 380P; or
h. the amino acids 30R, 74Q, 80P, 94L, 118L, 176P, 179K, 180N, 183A, 204N, 211V, 212G, 224E, 227E, 253Y, 276M, 287S, 315G, 352M, and 380P; or
i. the amino acids 30R, 94L, 118L, 126H, 176P, 179K, 180N, 204N, 211V, 212G, 224E, 253Y, 315G, 352M, and 380P; or
j. the amino acids 30R, 94L, 118L, 176P, 179K, 180N, 182K, 204N, 211V, 212G, 224E, 253Y, 315G, 352M, and 380P; or
k. the amino acids 30R, 74Q, 80P, 94L, 118L, 176P, 179K, 180N, 182K, 183A, 204N, 211V, 212G, 224E, 227E, 253Y, 276M, 287S, 315G, 352M, and 380P; or
I. the amino acids 30R, 35N, 74Q, 80P, 94L, 118L, 176P, 179K, 180N, 182K, 183A, 204N, 211V, 212G, 224E, 227E, 253Y, 276M, 287S, 315G, 352M, and 380P; or
m. the amino acids 30R, 35N, 74Q, 80P, 94L, 118L, 120R, 176P, 179K, 180N, 182K, 183A, 204N, 211V, 212G, 224E, 227E, 253Y, 276M, 287S, 315G, 352M, and 380P; or
n. the amino acids 30R, 31C, 74Q, 80P, 94L, 118L, 176P, 177C, 179K, 180N, 204N, 211V, 212G, 224E, 227E, 253Y, 276M, 287S, 315G, 352M, and 380P; or
o. the amino acids 30R, 35N, 74Q, 80P, 94L, 118L, 120R, 176P, 179K, 180N, 182K, 183A, 204N, 211V, 212G, 224E, 227E, 253Y, 276M, 287S, 315G, 352M, and 380P; or
p. the amino acids 30R, 80P, 94L, 118L, 140C, 176P, 179K, 180N, 200C, 204N, 211V, 212G, 224E, 227E, 253Y, 276M, 287S, 315G, 352M, and 380P; or
q. the amino acids 30R, 35N, 80P, 94L, 118L, 176P, 179K, 180N, 200I, 204N, 211V, 212G, 224E, 227E, 253Y, 276M, 287S, 315G, 352M, and 380P; or
r. the amino acids 30R, 56S, 671, 74Q, 94L, 107N, 118L, 154N, 174E, 176P, 179K, 180N, 202S, 204N, 211V, 212G, 224E, 253Y, 2711, 285E, 302A, 315G, 344D, 380P, and 395A; or
s. the amino acids 30R, 56S, 67I, 94L, 107N, 118L, 154N, 174E, 176P, 179K, 180N, 202S, 204N, 211V, 212G, 224E, 253Y, 2711, 285E, 302A, 315G, 344D, 352M, 380P, and 395A; or
t. the amino acids 30R, 56S, 67I, 74Q, 94L, 107N, 118L, 154N, 174E, 176P, 179K, 180N, 202S, 204N, 211V, 212G, 224E, 253Y, 2711, 285E, 302A, 315G, 344D, 352M, 380P, and 395A; or
u. the amino acids 30R, 56S, 67I, 74Q, 80P, 94L, 107N, 118L, 154N, 174E, 176P, 179K, 180N, 202S, 204N, 211V, 212G, 224E, 227E, 253Y, 2711, 276M, 285E, 287S, 302A, 315G, 344D, 352M, 380P, and 395A; or
v. the amino acids 30R, 56S, 67I, 80P, 94L, 107N, 118L, 154N, 174E, 176P, 179K, 180N, 202S, 204N, 211V, 212G, 224E, 227E, 253Y, 2711, 276M, 285E, 287S, 302A, 315G, 344D, 352M, 380P, and 395A; or
w. the amino acids 30R, 35N, 56S, 67I, 74Q, 80P, 94L, 107N, 118L, 154N, 174E, 176P, 179K, 180N, 202S, 204N, 211V, 212G, 224E, 227E, 253Y, 271I, 276M, 285E, 287S, 302A, 315G, 344D, 352M, 380P, and 395A; or
x. the amino acids 30R, 35N, 56S, 67I, 80P, 94L, 107N, 118L, 154N, 174E, 176P, 179K, 180N, 202S, 204N, 211V, 212G, 224E, 227E, 253Y, 2711, 276M, 285E, 287S, 302A, 315G, 344D, 352M, 380P, and 395A; or
y. the amino acids 30R, 35N, 56S, 67I, 80P, 94L, 107N, 118L, 120R, 154N, 174E, 176P, 179K, 180N, 202S, 204N, 211V, 212G, 224E, 227E, 253Y, 271I, 276M, 285E, 287S, 302A, 315G, 344D, 352M, 380P, and 395A; or
z. the amino acids 30R, 56S, 67I, 80P, 94L, 107N, 118L, 154N, 176P, 179K, 180N, 202S, 204N, 211V, 212G, 224E, 227E, 253Y, 2711, 276M, 285E, 287S, 302A, 315G, 344D, 352M, 380P, and 395A; or
aa.the amino acids 30R, 56S, 67I, 80P, 94L, 107N, 118L, 126H, 154N, 174E, 176P, 179K, 180N, 202S, 204N, 211V, 212G, 224E, 227E, 253Y, 2711, 276M, 285E, 287S, 302A, 315G, 344D, 352M, 380P, and 395A; or
bb.30R, 80P, 94L, 118L, 176P, 179K, 180N, 204N, 211V, 212G, 224E, 227E, 253Y, 276M, 277A, 287S, 315G, and 380P; or
cc. a set of amino acid substitutions specified in Table 1.

In an embodiment the variant polypeptide comprises the substitutions selected from:
a. Q30R, K74Q, R94L, T118L, N176P, L179K, K180N, D204N, W211V, S212G, Q224E, V253Y, E315G, and A380P; or
b. Q30R, R94L, T118L, N176P, L179K, K180N, D204N, W211V, S212G, Q224E, V253Y, E315G, L352M, and A380P; or
c. Q30R, K74Q, R94L, T118L, N176P, L179K, K180N, D204N, W211V, S212G, Q224E, V253Y, E315G, L352M, and A380P; or
d. Q30R, K74Q, S80P, R94L, T118L, N176P, L179K, K180N, D204N, W211V, S212G, Q224E, Q227E, V253Y, K276M, Q287S, E315G, L352M, and A380P; or
e. Q30R, D35N, S80P, R94L, T118L, N176P, L179K, K180N, D204N, W211V, S212G, Q224E, Q227E, V253Y, K276M, Q287S, E315G, L352M, and A380P; or
f. Q30R, S80P, R94L, T118L, N176P, L179K, K180N, K183A, D204N, W211V, S212G, Q224E, Q227E, V253Y, K276M, Q287S, E315G, L352M, and A380P; or
g. Q30R, S80P, R94L, T118L, N176P, L179K, K180N, D204N, W211V, S212G, Q224E, Q227E, V253Y, K276M, Q287S, E315G, L352M, and A380P; or
h. Q30R, K74Q, S80P, R94L, T118L, N176P, L179K, K180N, K183A, D204N, W211V, S212G, Q224E, Q227E, V253Y, K276M, Q287S, E315G, L352M, and A380P; or
i. Q30R, R94L, T118L, N126H, N176P, L179K, K180N, D204N, W211V, S212G, Q224E, V253Y, E315G, L352M, and A380P; or
j. Q30R, R94L, T118L, N176P, L179K, K180N, E182K, D204N, W211V, S212G, Q224E, V253Y, E315G, L352M, and A380P; or
k. Q30R, K74Q, S80P, R94L, T118L, N176P, L179K, K180N, E182K, K183A, D204N, W211V, S212G, Q224E, Q227E, V253Y, K276M, Q287S, E315G, L352M, and A380P; or
I. Q30R, D35N, K74Q, S80P, R94L, T118L, N176P, L179K, K180N, E182K, K183A, D204N, W211V, S212G, Q224E, Q227E, V253Y, K276M, Q287S, E315G, L352M, and A380P; or
m. Q30R, D35N, K74Q, S80P, R94L, T118L, S120R, N176P, L179K, K180N, E182K, K183A, D204N, W211V, S212G, Q224E, Q227E, V253Y, K276M, Q287S, E315G, L352M, and A380P; or
n. Q30R, D31C, K74Q, S80P, R94L, T118L, N176P, L177C, L179K, K180N, D204N, W211V, S212G, Q224E, Q227E, V253Y, K276M, Q287S, E315G, L352M, and A380P; or
o. Q30R, D35N, K74Q, S80P, R94L, T118L, S120R, N176P, L179K, K180N, E182K, K183A, D204N, W211V, S212G, Q224E, Q227E, V253Y, K276M, Q287S, E315G, L352M, and A380P; or
p. Q30R, S80P, R94L, T118L, V140C, N176P, L179K, K180N, V200C, D204N, W211V, S212G, Q224E, Q227E, V253Y, K276M, Q287S, E315G, L352M, and A380P; or
q. Q30R, D35N, S80P, R94L, T118L, N176P, L179K, K180N, V200I, D204N, W211V, S212G, Q224E, Q227E, V253Y, K276M, Q287S, E315G, L352M, and A380P; or
r. Q30R, A56S, V67I, K74Q, R94L, D107N, T118L, D154N, Q174E, N176P, L179K, K180N, A202S, D204N, W211V, S212G, Q224E, V253Y, L271I, Q285E, G302A, E315G, N344D, A380P, and G395A; or
s. Q30R, A56S, V67I, R94L, D107N, T118L, D154N, Q174E, N176P, L179K, K180N, A202S, D204N, W211V, S212G, Q224E, V253Y, L271I, Q285E, G302A, E315G, N344D, L352M, A380P, and G395A; or
t. Q30R, A56S, V67I, K74Q, R94L, D107N, T118L, D154N, Q174E, N176P, L179K, K180N, A202S, D204N, W211V, S212G, Q224E, V253Y, L271I, Q285E, G302A, E315G, N344D, L352M, A380P, and G395A; or
u. Q30R, A56S, V67I, K74Q, S80P, R94L, D107N, T118L, D154N, Q174E, N176P, L179K, K180N, A202S, D204N, W211V, S212G, Q224E, Q227E, V253Y, L271I, K276M, Q285E, Q287S, G302A, E315G, N344D, L352M, A380P, and G395A; or
v. Q30R, A56S, V67I, S80P, R94L, D107N, T118L, D154N, Q174E, N176P, L179K, K180N, A202S, D204N, W211V, S212G, Q224E, Q227E, V253Y, L271I, K276M, Q285E, Q287S, G302A, E315G, N344D, L352M, A380P, and G395A; or
w. Q30R, D35N, A56S, V67I, K74Q, S80P, R94L, D107N, T118L, D154N, Q174E, N176P, L179K, K180N, A202S, D204N, W211V, S212G, Q224E, Q227E, V253Y, L271I, K276M, Q285E, Q287S, G302A, E315G, N344D, L352M, A380P, and G395A; or
x. Q30R, D35N, A56S, V67I, S80P, R94L, D107N, T118L, D154N, Q174E, N176P, L179K, K180N, A202S, D204N, W211V, S212G, Q224E, Q227E, V253Y, L271I, K276M, Q285E, Q287S, G302A, E315G, N344D, L352M, A380P, and G395A; or
y. Q30R, D35N, A56S, V67I, S80P, R94L, D107N, T118L, S120R, D154N, Q174E, N176P, L179K, K180N, A202S, D204N, W211V, S212G, Q224E, Q227E, V253Y, L271I, K276M, Q285E, Q287S, G302A, E315G, N344D, L352M, A380P, and G395A; or
z. Q30R, A56S, V67I, S80P, R94L, D107N, T118L, D154N, N176P, L179K, K180N, A202S, D204N, W211V, S212G, Q224E, Q227E, V253Y, L271I, K276M, Q285E, Q287S, G302A, E315G, N344D, L352M, A380P, and G395A; or
aa.Q30R, A56S, V67I, S80P, R94L, D107N, T118L, N126H, D154N, Q174E, N176P, L179K, K180N, A202S, D204N, W211V, S212G, Q224E, Q227E, V253Y, L271I, K276M, Q285E, Q287S, G302A, E315G, N344D, L352M, A380P, and G395A;
bb. R94L and T118L/I; or
cc. Q174E, N176P, L179K, and K180N; or
dd.A202S and D204N; or
ee.A202S, D204N, S212G/A, and V253Y/Q; or
ff. L2711 and G302A; or
gg. Q285E and Q287S/K; or
hh. E315G and A380P; or
ii. Q30R, S80P, R94L, T118L, N176P, L179K, K180N, D204N, W211V, S212G, Q224E, Q227E, V253Y, K276M, T277A, Q287S, E315G, and A380P.

In an embodiment the variant polypeptide has an increased relative IP6 degrading activity compared to the SEQ ID NO: 1 when expressed in an expression host.

In another embodiment the present variant polypeptide may have an improved production yield when produced in an expression host cell.

The increased relative IP6 degrading activity means higher specific activity (more activity per mg phytase protein), a higher concentration of phytase in spent culture medium after cultivation, a more stable protein, or any combinations of these. The increased relative IP6 degrading activity of the present variant polypeptide can thus be used to an advantage for example in compositions which are challenged by demanding conditions that typically inactivate phytases.

In an embodiment the variant polypeptide has both an increased thermostability and an increased relative IP6 degrading activity compared to the SEQ ID NO: 1. The thermostability may be analysed according to Example 5 and the increased relative IP6 degrading activity may be analysed according to Example 4.

According to a second aspect is provided a recombinant host cell comprising genetic elements configured to produce at least one variant polypeptide, wherein the host cell is preferably selected from the group consisting of
filamentous fungal cells from Division Ascomycota, Subdivision Pezizomycotina; preferably from the group consisting of members of the Class Sordariomycetes or Eurotiomycetes, Subclass Hypocreomycetidae or Sordariomycetidae or Eurotiomycetidae, Orders Hypocreales or Sordariales or Eurotiales, Families Hypocreacea or Nectriacea or Chaetomiaceae or Aspergillaceae, Genera *Trichoderma* (anamorph of *Hypocrea*) or *Fusarium* or *Acremonium* or *Humicola* or *Thermothelomyces* or *Myceliophthora* or *Aspergillus;*
more preferably from the group consisting of species *Trichoderma reesei (Hypocrea jecorina), T. citrinoviridae, T. longibrachiatum, T. virens, T. harzianum, T. asperellum, T. atroviridae, T. parareesei, Fusarium oxysporum, F. gramineanum, F. pseudograminearum, F. venenatum, Acremonium (Cephalosporium) chrysogenum, Humicola insolens, H. grisea, Thermothelomyces thermophilus, Myceliophthora thermophila, Aspergillus niger, A. niger* var*. awamori and A. oryzae*;
bacterial cells, preferably gram-positive Bacilli such as *B*. *subtilis, B*. *licheniformis, B*. *megaterium, B*. *amyloliquefaciens, B*. *pumilus,* gram negative bacteria such as *Escherichia coli*, actinomycetales such as *Streptomyces* sp.; and
yeasts, such as *Saccharomyces cerevisiae, Schizosaccharomyces pombe, Pichia pastoris, Yarrowia lipolytica*; and
more preferably the host cell is selected from filamentous fungal cells such as Trichoderma, or from gram-positive Bacilli such as *Bacillus*;
most preferably from *Trichoderma reesei* or from *Bacillus subtilis* or *B*. *pumilus, B*. *licheniformis,* or *B*. *amyloliquefaciens*.

According to a third aspect is provided a recombinant host cell comprising genetic elements configured to produce at least one present variant polypeptide, and wherein the host cell is a transgenic plant cell.

According to a third aspect is provided an enzyme composition comprising the present variant polypeptide.

According to a fourth aspect is provided a use of the present variant polypeptide or the present enzyme composition in the manufacturing of feedstuff, foodstuff, feed additive, dietary supplement, or a pharmaceutical.

According to a fifth aspect is provided a method of manufacturing the present variant polypeptide comprising:
a.providing a polynucleotide comprising genetic elements arranged to produce the present variant polypeptide; and
b.expressing the polynucleotide in a recombinant host cell, preferably in the present recombinant host cell.

According to a sixth aspect is provided an animal feed comprising the present variant polypeptide, or the present enzyme composition, and at least one protein source of plant origin, and
a. optionally at least one further enzyme selected from protease, amylase, phytase, xylanase, endoglucanase, beta-glucanase, mannanase, cellulase, or a combination thereof; and
b. optionally at least one filler selected from maltodextrin, flour, salt, sodium chloride, sulphate, sodium sulphate, or a combination thereof.

According to a seventh aspect is provided a feed supplement comprising the present variant polypeptide or the present enzyme composition; and
a.optionally at least one further enzyme selected from protease, amylase, phytase, xylanase, endoglucanase, beta-glucanase, mannanase, or a combination thereof; and
b.optionally at least one filler selected from maltodextrin, flour, salt, sodium chloride, sulphate, sodium sulphate, minerals, amino acids, prebiotics, probiotics, or a combination thereof.

According to an eighth aspect is provided a method of degrading or modifying material containing phytic acid or phytate, comprising treating said material with an effective amount of the present variant polypeptide or the present enzyme composition.

### BRIEF DESCRIPTION OF THE FIGURES

Some example embodiments will be described with reference to the accompanying figures, in which:

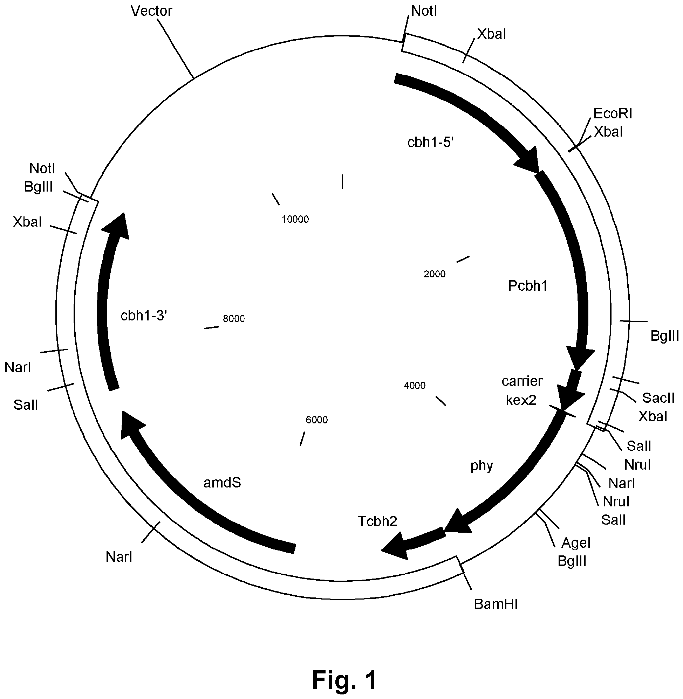

**Figure 1** shows a schematic picture of the expression plasmid used in the transformation of *Trichoderma reesei* for expression of parent phytase (SEQ ID NO:1) and phytase variant genes (phy). The expression of the recombinant genes in the host cell was controlled by use of the following genetic elements: *T. reesei cbh1* promoter (Pcbh1) for transcription initiation, and *T. reesei cbh2* (Tcbh2) terminator for transcription termination. *T. reesei cbh2* carrier encoding the CBHII CBM and linker region (carrier) was used instead of the native phytase signal sequence and Kex2 protease cleavage site (kex2) was included between the encoded carrier polypeptide and phytase. A synthetic gene (*amd*S) encoding the AmdS marker was included for selection of the transformants and *T. reesei cbh1* 3'- and 5'-flanking regions (cbh1-3' and cbh1-5', respectively) were used to optionally target the expression cassette to *cbh1* locus. The vector derives from pUC19. Picture was generated using Clone Manager Professional 9 from Sci-Ed Software. A selection of restriction enzyme sites is shown in the picture.

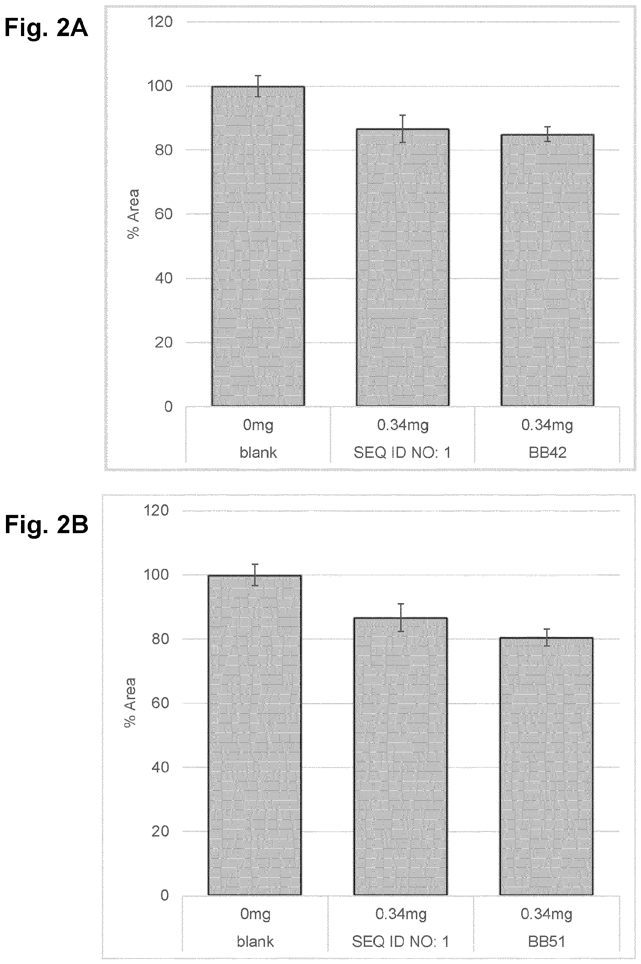

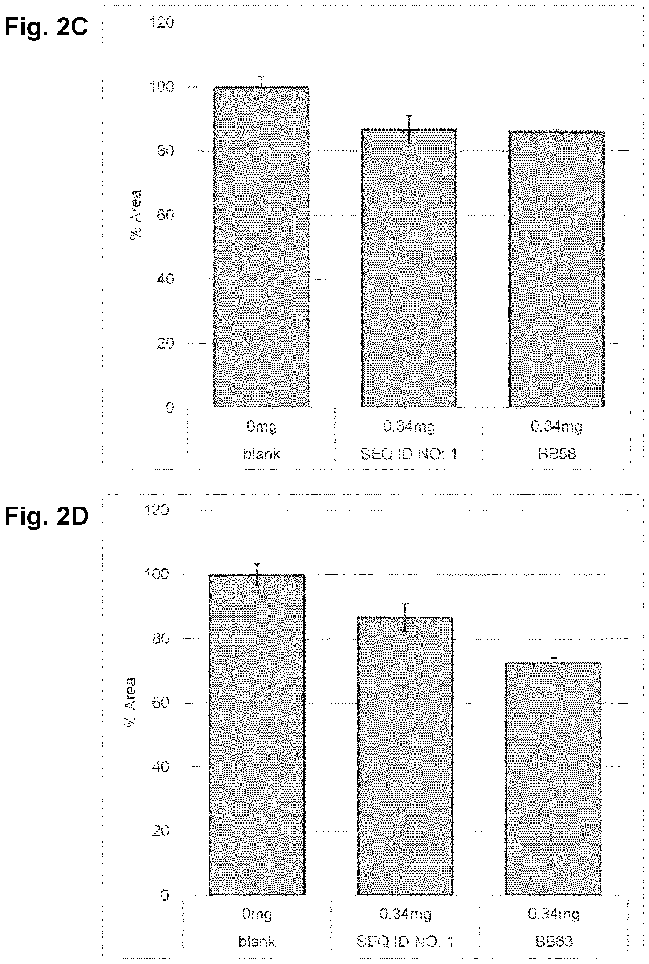

Figures 2A-2D outline the in vitro test (GIT) outcome for selected phytase variants with improved thermostability. The residual amount of the sum of IP6+IP5+IP4 after treatment of a corn-soybean meal-based feed with the tested phytase is compared to the residual amount of these inositolphosphates without any phytase (blank) or the same activity of phytase SEQ ID NO: 1 added. The dosing of the phytases was 34 mg of purified protein per kg feed mix.Fig 2A to D demonstrate an efficient degradation of the sum of IP6 to IP4 by phytase variants BB42 (Fig. 2 A); BB51 (Fig. 2 B); BB58 (Fig. 2 C) and BB63 (Fig. 2 D) compared to phytase SEQ ID No:1.
**Fig. 2A** demonstrates a comparable degradation of the sum of IP6 to IP4 compared to SEQ ID No: 1 phytase by variant BB42 after in vitro treatment (GIT) of a corn-soybean based feed when added as purified protein with 34 mg/kg.
**Fig. 2B** demonstrates a slightly further degradation of the sum of IP6 to IP4 compared to SEQ ID No: 1 phytase by variant BB51 after in vitro treatment (GIT) of a corn-soybean based feed when added as purified protein with 34 mg/kg.
**Fig. 2C** demonstrates a comparable degradation of the sum of IP6 to IP4 compared to SEQ ID No: 1 phytase by variant BB58 after in vitro treatment (GIT) of a corn-soybean based feed when added as purified protein with 34 mg/kg.
**Fig. 2D** demonstrates further degradation of the sum of IP6 to IP4 compared to SEQ ID No: 1 phytase by variant BB63 after in vitro treatment (GIT) of a corn-soybean based feed when added as purified protein with 34 mg/kg.
**Figure 3** Phytase recovery in feed after conditioning (30 sec) at 80°C or 95°C followed by pelleting (data given relative to the analysed enzyme activity in mash feed; %). The BB19 variant showed an increased thermostability compared to SEQ ID NO: 1.
**Figure 4A-4F** outline the feeding trials. Performance evaluation, in each trial compared to birds being fed the corresponding basal diet that was reduced in phosphorus (P) but without any phytase, demonstrate the efficiency of these variants. Data is shown separately for the trials in Fig. 4A and B (trial 1), Fig 4C and D (trial 2) and Fig 4E and F (trial 3). Broiler performance was improved by all variants demonstrating the efficiency of the phytases to release phytate bound phosphorus in broilers.

In all trials broilers were fed corn soybean based, mash diets, in trial 1 and trial 3 added by 8-10% rape seed meal also.

While BB16 and BB19 were applied to broilers in a 2-phase feeding program from day 1-35 in both trials to demonstrate the efficacy of variants, BB58 and BB63 were fed in one feeding phase to broilers from day 1 to 21.

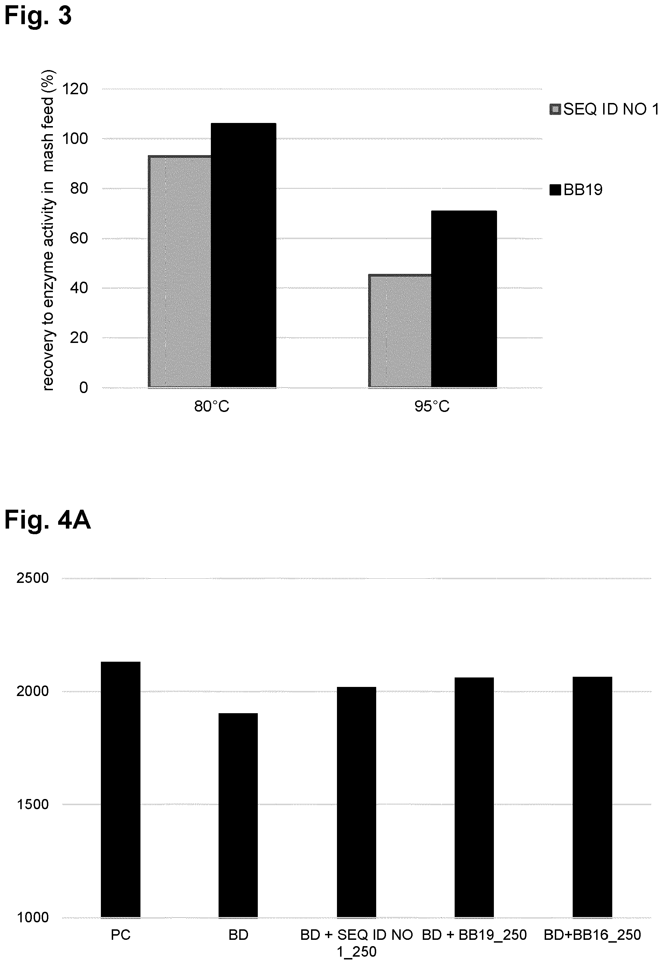

**Fig. 4A**: Body weight of 35 day (d) old broilers in grams (g). PC = positive control with recommended P and Ca level; BD = basal diet with reduced P and Ca level. Phytase fed birds received BD diets added with 250 FTU/kg feed.

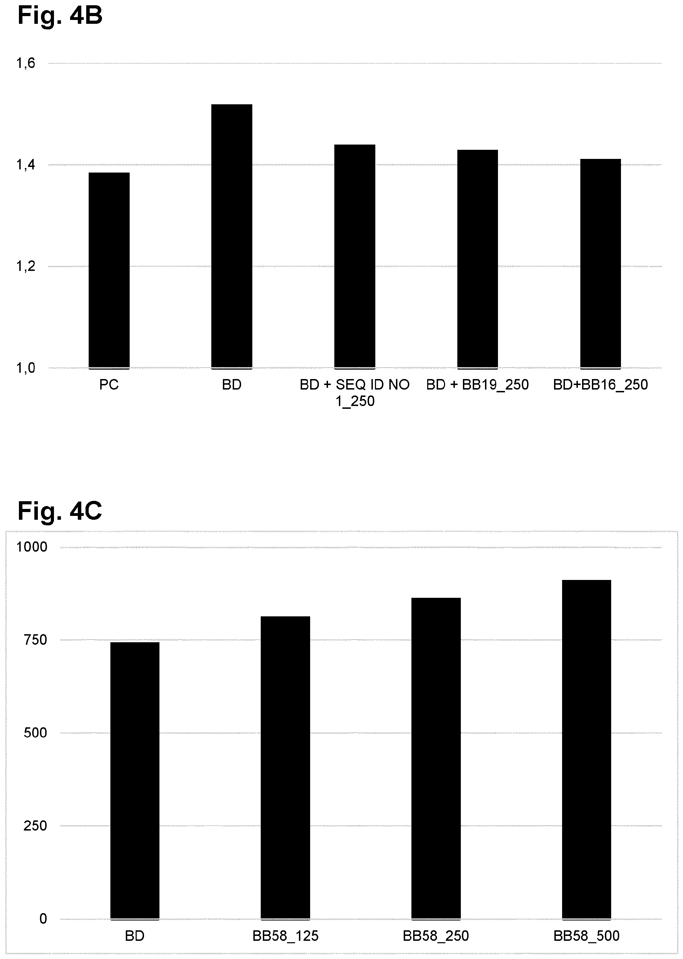

**Fig. 4B**: Feed conversion ratio (FCR) of 35 day (d) broilers in grams (g) / g. PC = positive control with recommended P and Ca level; BD = basal diet with reduced P and Ca level. Phytase fed birds received BD diets added with 250 FTU/kg feed.

**Fig. 4C**: Body weight of 21 day (d) old broilers in grams (g). BD = basal diet with reduced P and Ca level. Phytase fed birds received BD diets added with 125, 250 or 500 FTU/kg feed.

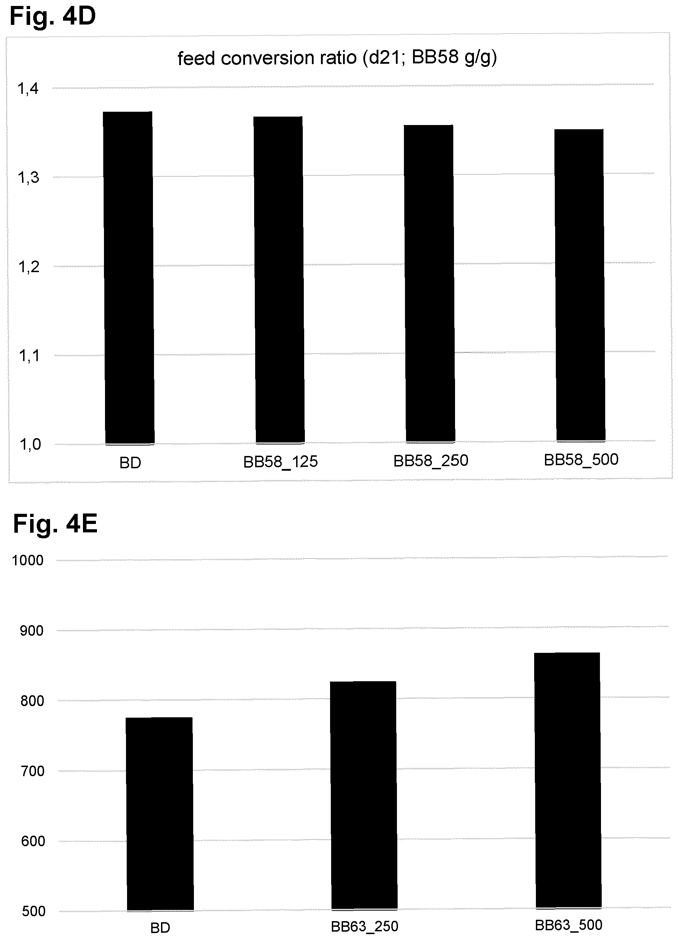

**Fig. 4D**: Feed conversion ratio (FCR) of 21 day (d) old broilers in grams (g) / g. BD = basal diet with reduced P and Ca level. Phytase fed bird received BD diets added with 125, 250 or FTU/kg feed.

**Fig. 4E****:** Body weight of 21 day (d) old broilers in grams (g). BD = basal diet with reduced P and Ca level. Phytase fed bird received BD diets added with 250 or 500 FTU/kg phytase.

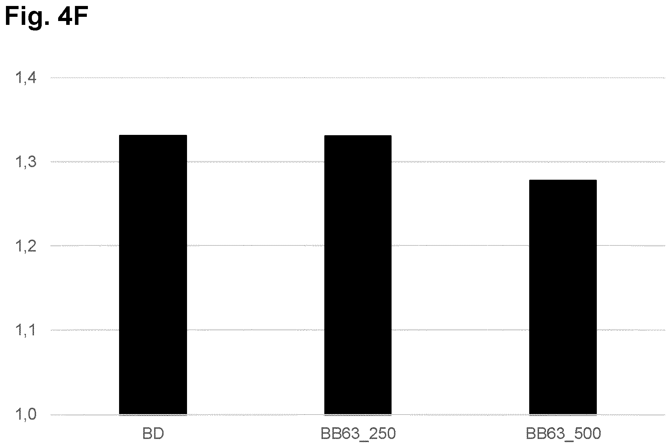

**Fig. 4F****:** Feed conversion ratio (FCR) of 21 day (d) old broilers in grams (g) / g. BD = basal diet with reduced P and Ca level. Phytase fed bird received BD diets added with 250 or 500 FTU/kg phytase.

### SEQUENCE LISTING

SEQ ID NO: 1: Amino acid sequence of the parent phytase without signal peptide.

### DETAILED DESCRIPTION

As used herein, the term "phytase" means an enzyme having capability to enzymatically degrade phytic acid to lower inositol phosphates.

Phytases are classified into 3-, 5- or 6-phytases (EC 3.1.3.8, EC 3.1.3.72 and EC 3.1.3.26, respectively) based on the carbon position on the inositol ring at which they preferably initiate phosphate hydrolysis. 6-phytases preferably first remove the phosphate group at the C6 position.

In an embodiment the polypeptide comprising the phytase variant comprises at least one further amino acid sequence selected from a signal sequence, a secretory sequence, a carrier polypeptide, a binding domain, a tag, an enzyme, a linker, or any combination thereof.

The terms "phytase variant", "variant of phytase", or "variant polypeptide" mean a phytase molecule obtained by site-directed or random mutagenesis, insertion, substitution, deletion, recombination and/or any other protein engineering method, and which leads into a genetically modified phytase that differs in its amino acid sequence from the parent phytase such as a wild type phytase. The terms "wild type phytase ", "wild type enzyme", "wild type", or "wt" in accordance with the disclosure, describe a phytase enzyme with an amino acid sequence found in nature or a fragment thereof. The variant encoding gene can be synthetised or the parent gene be modified using genetic methods, e.g., by site-directed mutagenesis, a technique in which one or more than one mutation is introduced at one or more defined sites in a polynucleotide encoding the parent polypeptide. The term variant phytase may also be referred to by using a name given to the variant in the examples and in the tables.

As used herein, the term "mature polypeptide" means any polypeptide wherein at least one signal sequence or signal peptide or signal peptide and a putative pro-peptide, or a carrier peptide or a fusion partner is cleaved off.

The term carrier polypeptide or fusion partner refers to a polypeptide into which the protein of interest (phytase) is translationally fused to improve the yield. The carrier/fusion partner can be either homologous or heterologous to production host in its origin and can be a full-length protein or a fragment of a protein (e.g. a core, a CBM or a CBM and linker region). It is preferably encoded by a gene or a nucleotide sequence with good expression level.

As used herein, a "peptide" and a "polypeptide" are amino acid sequences including a plurality of consecutive polymerized amino acid residues. For purpose of this disclosure, peptides are molecules including up to 20 amino acid residues, and polypeptides include more than 20 amino acid residues. The peptide or polypeptide may include modified amino acid residues, naturally occurring amino acid residues not encoded by a codon, and non-naturally occurring amino acid residues. As used herein, a "protein" may refer to a peptide or a polypeptide of any size. A protein may be an enzyme, a protein, an antibody, a membrane protein, a peptide hormone, regulator, or any other protein.

As used herein, "sequence identity" means the percentage of exact matches of amino acid residues between two optimally aligned sequences over the number of positions where there are residues present in both sequences. When one sequence has a residue with no corresponding residue in the other sequence, the alignment program allows a gap in the alignment, and that position is not counted in the denominator of the identity calculation.

As used herein, "sequence alignment" of the amino acid sequences means, aligning the sequences using Clustal Omega (1.2.4) multiple sequence alignment program (https://www.ebi.ac.uk/Tools/msa/clustalo/) as described by Sievers et al 2011, and using the default settings.

Unless otherwise specified, all references to a certain amino acid position refer to an amino acid of the SEQ ID NO: 1 in said position, or to an amino acid present or missing in the corresponding position of an amino acid sequence aligned with SEQ ID NO: 1.

As used herein, the term "corresponding positions" or "corresponding amino acid position" means aligning at least two amino acid sequences according to identified regions of similarity or identity as pairwise alignment or as multiple sequence alignment, thereby pairing up the corresponding amino acids. In the present disclosure corresponding positions typically refers to a position corresponding to the position in SEQ ID NO: 1.

As used herein, "amino acid substitution" means an amino acid residue replacement with an amino acid residue that is different than the original amino acid in that specific replacement position. The term "amino acid substitution" can refer to conservative amino acid substitutions and non-conservative amino acid substitutions, which means the amino acid residue is replaced with an amino acid residue having a similar side chain (conservative), or a different side chain (non-conservative), as the original amino acid residue in that place.

The term "functional fragment" means a fragment or portion of the current variant, which retains about the same enzymatic function or effect.

The term "secretory signal sequence" or "signal sequence" or a "secretory peptide" refers to an amino acid sequence which is a component or a part of a larger polypeptide, and which directs the larger polypeptide through a secretory pathway of a host cell in which it is produced. The secretory signal sequence can be native or it can be obtained from another source. Depending on the host cell, the larger polypeptide may be cleaved from the secretory peptide during transit through the secretory pathway, thereby forming a mature polypeptide lacking the secretory peptide.

"Phytase activity" as used herein, refers to the phytic acid degrading activity. Example 4 provides examples of a method for determining phytase activity.

In an embodiment the term "enzyme composition" means an enzymatic fermentation product, possibly isolated and purified, typically produced by a pure culture of a microorganism. The enzyme composition usually comprises a number of different enzymatic activities produced by the microorganism. In another embodiment the enzyme composition is a mixture of monocomponent enzymes, preferably enzymes derived from bacterial or fungal species by using conventional recombinant production techniques. The enzyme composition may contain for example stabilators or preservatives which prevent microbial growth and improves storage stability.

As used herein, a "host cell" means any cell type that is susceptible to transformation, transfection, transduction, mating, crossing or the like with a nucleic acid construct or expression vector comprising a polynucleotide. The term "host cell" encompasses any progeny that is not identical due to mutations that occur during replication. Non-limiting examples of a host cell are fungal cells, preferably a filamentous fungal cell (e.g. *Trichoderma* or *Trichoderma reesei, Aspergillus* or *Aspergillus oryzae* or *Aspergillus niger, Thermothelomyces or Thermothelomyces heterothallica, Myceliophthora* or *Myceliophthora thermophila*, or *Humicola or Humicola insolens* or *Fusarium* or *Fusarium venenatum*₎, bacterial cells, preferably gram-positive Bacilli (e.g., *Bacillus subtilis*, *B*. *licheniformis*, *B*. *megaterium*, *B. amyloliquefaciens, B*. *pumilus),* gram-negative bacteria (e.g., *Escherichia coli*), actinomycetales (e.g., *Streptomyces sp., Nonomuraea flexuosa*) and yeasts (e.g., *Saccharomyces cerevisiae*, *Schizosaccharomyces pombe, Pichia pastoris, Yarrowia lipolytica).*

In another embodiment the phytase variant is obtained by recombinant production in plant cells, i.e., in a transgenic plant.

The recombinant host cell can be used to produce the phytase variant and to contain a polynucleotide encoding it. The recombinant host cell can be operably linked to one or more control sequences that direct the production of the variant, and that make it possible to initiate the production of the present phytase variant by a stimulus, as is known in the field. The recombinant host cell is useful also in preparation of variants with different properties. For example, a host cell can be selected, which provides post-translational modifications beneficial for stability or activity, or which facilitates post-processing of a variant produced in the host cell.

In an embodiment the host cell is non-pathogenic. This is particularly advantageous for using the host cell or the phytase variant produced in it for animal feed.

In an embodiment the composition containing the phytase variant is food or feed, and it may further comprise plant material which contains phytic acid.

In an embodiment the composition is a food additive or a feed additive further comprising at least one of: at least one trace mineral, at least one amino acid, in particular lysine, water soluble vitamin, fat soluble vitamin, prebiotic, and probiotic.

In an embodiment the composition is a food additive or a feed additive complying with the requirements of Regulation (EC) No 1831/2003 of the European Parliament and of the Council of 22 September 2003 on additives for use in animal nutrition.

In an embodiment the composition is in a form of a liquid composition or a solid composition such as solution, dispersion, paste, pellet, powder, granule, coated granule, tablet, cake, crystal, crystal slurry, gel, extrude, precipitate, premix, or a combination thereof.

The term "stability" refers to the stability of the phytase variant as a function of time in a certain environmental condition. Different methods are used to analyze the stability of phytase variants. The unfolding temperature of a phytase variant can be measured to assess the thermostability of the phytase variant, as described in the Example 5. The residual enzyme activity can be measured by using the "activity assay" as described in the Examples. The term "stability" may include stability during use in a process with high temperature conditions and/or during storage and/or stability against proteases.

The term "promoter" refers to a portion of a gene containing DNA sequence(s) that provide for the binding of RNA polymerase and initiation of transcription. Promoter sequences are commonly, but not always, found in the 5' non-coding regions of genes. As used herein, the terms "domain" and "region" can be used interchangeably with the term "module".

The following abbreviations are used for amino acids:

| | | |
|---|---|---|
| A | Ala | Alanine |
| C | Cys | Cysteine |
| D | Asp | Aspartic acid |
| E | Glu | Glutamic acid |
| F | Phe | Phenylalanine |
| G | Gly | Glycine |
| H | His | Histidine |
| I | Ile | Isoleucine |
| K | Lys | Lysine |
| L | Leu | Leucine |
| M | Met | Methionine |
| N | Asn | Asparagine |
| P | Pro | Proline |
| Q | Gln | Glutamine |
| R | Arg | Arginine |
| S | Ser | Serine |
| T | Thr | Threonine |
| V | Val | Valine |
| W | Trp | Tryptophan |
| Y | Tyr | Tyrosine |
| X | Xaa | Any one of the above amino acids |

Substitutions are described herein by using of the following nomenclature: amino acid residue in the protein scaffold, i.e., the parent sequence; position; substituted amino acid residue(s). According to this nomenclature the substitution of, for instance, a single residue of alanine to tyrosine residue at position 23 is indicated as Ala23Tyr or A23Y. A substitution of any amino acid in position 23 to tyrosine is indicated as Xaa23Tyr or X23Y or 23Y. A substitution of a tyrosine in position 179 to phenylalanine, tryptophan, or leucine is indicated as Y179F/W/L.

As used herein, the term "comprising" includes the broader meanings of "including", "containing", and "comprehending", as well as the narrower expressions "consisting of" and "consisting only of".

As used herein, the term "expression" includes any step or all steps involved in the production of a polypeptide in a host cell including, but not limited to, transcription, translation, post-translational modification, and secretion. Expression may be followed by harvesting, i.e., recovering, the host cells or the expressed product.

In an embodiment the phytase variant has phytase activity, and an amino acid sequence with at least 80 %, at least 85 %, at least 90 %, at least 92%, at least 94%, at least 95 %, at least 96%, at least 97%, at least 98%, or at least 99% sequence identity with amino acids of SEQ ID NO: 1. In an embodiment the variant polypeptide does not have 100% sequence identity with amino acids of SEQ ID NO: 1. In an embodiment, the amino acid numbering of the variant polypeptide corresponds to that of SEQ ID NO: 1. In an alternative embodiment, the amino acid numbering of the variant polypeptide corresponds to that of SEQ ID NO: 1 partially.

In an embodiment the total number of the amino acid substitutions in the variant polypeptide, compared to the SEQ ID NO: 1, is at least 2. The at least one further amino acid may be selected from the positions disclosed herein. In another embodiment the total number of substitutions is at least 5, at least 10, at least 15, at least 20 or at least 25; or 5, 10, 15, 20, or 25. In an embodiment the substitution or the substitutions are made in the non-conservative region. In another embodiment the variant polypeptide comprises the substitutions specified in any claim, and additional substitutions in non-conserved region of the amino acid-sequence. The effect these additional substitutions have on the properties can be analyzed as described in the Examples.

In an embodiment the variant polypeptide, or the functional fragment, has a predicted molecular weight between 40 and 60 and kDa, preferably between 43-55 kDa. The predicted molecular weight can be determined by calculating the sum of the molecular weights of the individual amino acids in the variant polypeptide, or in its functional fragment. When the predicted molecular weight of the variant polypeptide is within the above range, the structure of the variant polypeptide may be similar with the parent sequence to which the substitutions are made.

Providing phytase variants that retain stability in high temperatures is advantageous for applications wherein the phytase need to survive from exposure to high temperature. High temperature tolerance of phytase variants may have other benefits like increased specific activity, increased enzyme storage stability or improved stability against proteases.

In an embodiment the unfolding temperature of the variant polypeptide is improved, i.e. it is higher, compared to SEQ ID NO: 1. The unfolding temperature can be determined as described in Example 5.

In an embodiment the unfolding temperature of the variant polypeptide is at least 0.1, 0.2, 0.3, 0.4, 0.5, 0.6, 0.7, 0.8, 0.9 or 1°C higher than that of the SEQ ID NO: 1. In another embodiment the unfolding temperature of the variant polypeptide is at least 0.5, 1, 1.5, 2, 2.5, 3, 3.5, or 4°C higher than that of the SEQ ID NO: 1. In another embodiment the unfolding temperature of the variant polypeptide is improved at least 1 or 1.5°C higher than that of SEQ ID NO: 1.

In an embodiment the unfolding temperature of the variant polypeptide is more than 88.7°C. In another embodiment the unfolding temperature of the variant polypeptide is at least 89 or 89.0°C.

In an embodiment the composition is provided in the form of a liquid composition or a solid composition, such as solution, dispersion, paste, powder, granule, granulate, coated granulate, tablet, cake, crystal, crystal slurry, gel, or pellet.

In an embodiment the phytic acid is degraded in a plant-based material or partly plant based material which contains phytic acid.

In an embodiment the present phytase variant is used in an animal feed, and the animal is a ruminant or a non-ruminant. In another embodiment the animal is a cattle like beef or cow, a sheep or goat. In another embodiment the non-ruminant include poultry (such as broiler, layer and turkey and duck); pigs (such as piglets, growing pigs and sows); fish (such as salmonids, carp, tilapia and catfish) and crustaceans.

In an embodiment the feed is animal feed intended to be fed to animals such as any compound feed or mixture. In another embodiment feed comprises or consists of grains such as maize, wheat, oats, barley, sorghum and rice; protein sources like soybean meal, sunflower meal and canola meal as well as of minerals.The feed, wherein the present variant is used, has improved nutritional value compared to a feed without the variant. The present composition and the present phytase variant degrade phytic acid of the feed and thereby increase its nutritional value for the animals. The animal feed, wherein the present phytase variant or the present composition is used, can be formulated in the form of a wet composition or a dry composition.

### EXAMPLES

The present invention is further disclosed by the following non-limiting examples.

### Example 1. Computational design of variants with improved thermal stability

The crystal structure of E. coli 6-phytase was obtained from the PDB database (1DKP). All atoms not part of the protein chain were removed and hydrogens were added according to a pH of 5.0. A rectangular simulation box was added with a minimal distance of 10 Angstroms to the protein atoms and subsequently filled with water molecules and ions to yield a solvated and neutralized system. To obtain an equilibrated system at room temperature, atom velocities were assigned from a Boltzmann distribution according to 300 K and the system was equilibrated coupled to a heat bath and a barostat at a temperature of 300 K and a pressure of 1.0 bar (system EQ300). To obtain an additional system equilibrated at a high temperature, the pressure coupling was removed, new atom velocities were drawn from a Boltzmann distribution according to 400 K and the system was equilibrated coupled to a heat bath at a temperature of 400 K (system EQ400).

For the system equilibrated at 300 K (EQ300) as well as for the system equilibrated at 400 K (EQ400), multiple production simulation runs with newly assigned particle velocities drawn from a Boltzmann distribution according to the respective temperatures were conducted. For both systems, the individual trajectories of the production runs were combined and subsequently analyzed. The root mean square fluctuation (RMSF) was calculated for all Cα atoms over all frames of the combined trajectory at 300K (RMSFCα-300K) as well as for all Cα atoms over all frames of the combined trajectory at 400K (RMSFCα-400K). The difference of the RMSF at both temperatures was calculated as ΔRMSFCα = RMSFCα-400K - RMSFCα-300K. To obtain representative conformations of the production simulations, both combined trajectories were clustered by the Cα root mean square deviation (RMSD). Mean Cα RMSD values (RMSDCα-mean) were then calculated between all conformations of the largest clusters of the combined trajectory at 400 K and all conformations of the largest clusters of the combined trajectory at 300 K for all Cα atoms. A set of temperature sensitive positions with either a high ΔRMSFCα or a high RMSDCα-mean value was derived by assessment of the analysis results. All temperature sensitive positions as well as positions in close proximity to temperature sensitive positions were subsequently analyzed by geometrical criteria to derive candidates for amino acid substitutions with a potentially stabilizing effect (substitution candidates). For each of the thus derived substitution candidates, a system was constructed, equilibrated, simulated and analyzed as described above to yield ΔRMSFCα and RMSDCα-mean values. Substitution candidates were ranked by their improvement in conformational stability according to ΔRMSFCα and RMSDCα-mean values and the most promising candidates were selected for experimental characterization.

The designed phytase variants are described in detail in Table 1.

**Table 1. List of phytase variants designed. The amino acid numbering corresponds to the amino acid numbering of the mature parent phytase molecule presented in SEQ ID NO: 1.**

| **Variant code** | **Mutation(s)** |
|---|---|
| 00161 | R94L |
| 00173 | Q224E |
| 00928 | Q30R, D35N, A87T, R94L, T118L, P121K, N126D, K180N, E182K, D204N, W211V, S212G, M216T, Q224E, V253Y, E315G, L352M, A380P |
| 00933 | Q30R, D35N, A87T, R94L, T118L, P121K, N126D, E182K, E186K, D204N, W211V, S212G, M216T, Q224E, V253Y, E315G, L352M, A380P |
| 00937 | Q30R, A87T, R94L, T118L, P121K, N126D, K180N, E182K, E186K, D204N, W211V, S212G, M216T, Q224E, V253Y, E315G, L352M, A380P |
| BB10 | Q30R, T93C, R94L, T118L, K180N, D204N, W211V, S212A, Q224E, Q225E, V253Y, E315G, Y327T, A380P |
| BB16 | Q30R, R94L, T118L, N176P, L179K, K180N, D204N, W211V, S212G, Q224E, V253Y, E315G, A380P |
| BB19 | Q30R, S80P, R94L, T118L, N176P, L179K, K180N, D204N, W211V, S212G, Q224E, Q227E, V253Y, K276M, T277A, Q287S, E315G, A380P |
| BB37 | Q30R, K74Q, R94L, T118L, N176P, L179K, K180N, D204N, W211V, S212G, Q224E, V253Y, E315G, A380P |
| BB38 | Q30R, R94L, T118L, N176P, L179K, K180N, D204N, W211V, S212G, Q224E, V253Y, E315G, L352M, A380P |
| BB39 | Q30R, K74Q, R94L, T118L, N176P, L179K, K180N, D204N, W211V, S212G, Q224E, V253Y, E315G, L352M, A380P |
| BB40 | Q30R, K74Q, S80P, R94L, T118L, N176P, L179K, K180N, D204N, W211V, S212G, Q224E, Q227E, V253Y, K276M, Q287S, E315G, L352M, A380P |
| BB41 | Q30R, D35N, S80P, R94L, T118L, N176P, L179K, K180N, D204N, W211V, S212G, Q224E, Q227E, V253Y, K276M, Q287S, E315G, L352M, A380P |
| BB42 | Q30R, S80P, R94L, T118L, N176P, L179K, K180N, K183A, D204N, W211V, S212G, Q224E, Q227E, V253Y, K276M, Q287S, E315G, L352M, A380P |
| BB43 | Q30R, S80P, R94L, T118L, N176P, L179K, K180N, D204N, W211V, S212G, Q224E, Q227E, V253Y, K276M, Q287S, E315G, L352M, A380P |
| BB44 | Q30R, K74Q, S80P, R94L, T118L, N176P, L179K, K180N, K183A, D204N, W211V, S212G, Q224E, Q227E, V253Y, K276M, Q287S, E315G, L352M, A380P |
| BB45 | Q30R, R94L, T118L, N126H, N176P, L179K, K180N, D204N, W211V, S212G, Q224E, V253Y, E315G, L352M, A380P |
| BB46 | Q30R, R94L, T118L, N176P, L179K, K180N, E182K, D204N, W211V, S212G, Q224E, V253Y, E315G, L352M, A380P |
| BB47 | Q30R, K74Q, S80P, R94L, T118L, N176P, L179K, K180N, E182K, K183A, D204N, W211V, S212G, Q224E, Q227E, V253Y, K276M, Q287S, E315G, L352M, A380P |
| BB48 | Q30R, D35N, K74Q, S80P, R94L, T118L, N176P, L179K, K180N, E182K, K183A, D204N, W211V, S212G, Q224E, Q227E, V253Y, K276M, Q287S, E315G, L352M, A380P |
| BB49 | Q30R, D35N, K74Q, S80P, R94L, T118L, S120R, N176P, L179K, K180N, E182K, K183A, D204N, W211V, S212G, Q224E, Q227E, V253Y, K276M, Q287S, E315G, L352M, A380P |
| BB50 | Q30R, D31C, K74Q, S80P, R94L, T118L, N176P, L177C, L179K, K180N, D204N, W211V, S212G, Q224E, Q227E, V253Y, K276M, Q287S, E315G, L352M, A380P |
| BB51 | Q30R, D35N, K74Q, S80P, R94L, T118L, S120R, N126H, N176P, L179K, K180N, E182K, K183A, D204N, W211V, S212G, Q224E, Q227E, V253Y, K276M, Q287S, E315G, L352M, A380P |
| BB52 | Q30R, S80P, R94L, T118L, V140C, N176P, L179K, K180N, V200C, D204N, W211V, S212G, Q224E, Q227E, V253Y, K276M, Q287S, E315G, L352M, A380P |
| BB53 | Q30R, D35N, S80P, R94L, T118L, N176P, L179K, K180N, V200I, D204N, W211V, S212G, Q224E, Q227E, V253Y, K276M, Q287S, E315G, L352M, A380P |
| BB54 | Q30R, A56S, V67I, K74Q, R94L, D107N, T118L, D154N, Q174E, N176P, L179K, K180N, A202S, D204N, W211V, S212G, Q224E, V253Y, L271I, Q285E, G302A, E315G, N344D, A380P, G395A |
| BB55 | Q30R, A56S, V67I, R94L, D107N, T118L, D154N, Q174E, N176P, L179K, K180N, A202S, D204N, W211V, S212G, Q224E, V253Y, L271I, Q285E, G302A, E315G, N344D, L352M, A380P, G395A |
| BB56 | Q30R, A56S, V67I, K74Q, R94L, D107N, T118L, D154N, Q174E, N176P, L179K, K180N, A202S, D204N, W211V, S212G, Q224E, V253Y, L271I, Q285E, G302A, E315G, N344D, L352M, A380P, G395A |
| BB57 | Q30R, A56S, V67I, K74Q, S80P, R94L, D107N, T118L, D154N, Q174E, N176P, L179K, K180N, A202S, D204N, W211V, S212G, Q224E, Q227E, V253Y, L271I, K276M, Q285E, Q287S, G302A, E315G, N344D, L352M, A380P, G395A |
| BB58 | Q30R, A56S, V67I, S80P, R94L, D107N, T118L, D154N, Q174E, N176P, L179K, K180N, A202S, D204N, W211V, S212G, Q224E, Q227E, V253Y, L271I, K276M, Q285E, Q287S, G302A, E315G, N344D, L352M, A380P, G395A |
| BB59 | Q30R, D35N, A56S, V67I, K74Q, S80P, R94L, D107N, T118L, D154N, Q174E, N176P, L179K, K180N, A202S, D204N, W211V, S212G, Q224E, Q227E, V253Y, L271I, K276M, Q285E, Q287S, G302A, E315G, N344D, L352M, A380P, G395A |
| BB60 | Q30R, D35N, A56S, V67I, S80P, R94L, D107N, T118L, D154N, Q174E, N176P, L179K, K180N, A202S, D204N, W211V, S212G, Q224E, Q227E, V253Y, L271I, K276M, Q285E, Q287S, G302A, E315G, N344D, L352M, A380P, G395A |
| BB61 | Q30R, D35N, A56S, V67I, S80P, R94L, D107N, T118L, S120R, D154N, Q174E, N176P, L179K, K180N, A202S, D204N, W211V, S212G, Q224E, Q227E, V253Y, L271I, K276M, Q285E, Q287S, G302A, E315G, N344D, L352M, A380P, G395A |
| BB62 | Q30R, A56S, V67I, S80P, R94L, D107N, T118L, D154N, N176P, L179K, K180N, A202S, D204N, W211V, S212G, Q224E, Q227E, V253Y, L271I, K276M, Q285E, Q287S, G302A, E315G, N344D, L352M, A380P, G395A |
| BB63 | Q30R, A56S, V67I, S80P, R94L, D107N, T118L, N126H, D154N, Q174E, N176P, L179K, K180N, A202S, D204N, W211V, S212G, Q224E, Q227E, V253Y, L271I, K276M, Q285E, Q287S, G302A, E315G, N344D, L352M, A380P, G395A |

### Example 2. Production of phytase variants

Standard molecular biology methods were used in the isolation and enzyme treatments of DNA (e.g., isolation of plasmid DNA, digestion of DNA to produce DNA fragments), in E. coli transformations, sequencing etc. The basic laboratory methods used were either as described by the enzyme, reagent or kit manufacturer or as described in the standard molecular biology handbooks, e.g. Sambrook and Russell (2001) or as described in the following examples.

The phytase genes encoding the designed phytase variants (Example 1, Table 1) were ordered as synthetic genes using codons optimised for expression in *Trichoderma reesei.* The codon optimised gene encoding the mature parent phytase was used as backbone for the synthetic genes encoding the variant phytases.

The phytases in the constructions were expressed from *T. reesei cbh1* (ce/7A) promoter using a carrier polypeptide (CBM and linker) encoding sequence from *T. reesei cbh2* (*cel6*A). A Kex2 protease cleavage site was included between the carrier polypeptide and phytase like described in Paloheimo et al., 2003. The transcription was terminated using cbh2 terminator, followed in the construction by a synthetic *amd*S marker gene. In addition, the constructions contain *cbh1* 3' and 5' flanking regions for optionally targeting the expression vector into the *cbh1* locus (Figure 1).

Circular expression plasmids were transformed in *T. reesei* protoplasts. The transformants were selected on plates containing acetamide as the sole nitrogen source. The host strain used lacks the four major endogenous *T*. *reesei* cellulases: CBHI/Cel7A, CBHII/Cel6A, EGI/Cel7B and EGII/Cel5A. The transformations were performed according to Penttilä et al, 1987, with the modifications described in Karhunen et al., 1993. Alternatively, CRISPR-Cas technology can be used in transformations.

The transformants were sporulated on potato dextrose agar (PDA) prior to cultivation.

The transformants were cultivated on 96-well plates (Havukainen et al, 2020) to analyse the phytase production of the transformants. Phytase activity of the recombinant variant phytases was measured from the culture supernatants as release of inorganic phosphate from sodium phytate as described in Example 4. Production of the recombinant protein was also detected from the culture supernatant by sodium dodecyl sulfate polyacrylamide gel electrophoresis (SDS-PAGE). The unfolding temperatures of phytases was analysed using Prometheus NT.48 equipment (NanoTemper GmbH, Munich, Germany) like described in Example 5.

All the transformants produced phytase activity. Transformants producing the most thermostable variant phytases and the reference strain producing the parent phytase were purified on selection plates through single conidia prior to sporulating them on PDA. The purified, selected transformants were cultivated in shake flasks and/or bioreactors in complex cellulase-inducing medium to obtain material for further characterisation and application tests.

### Example 3. Purification of phytases

Chosen phytases were purified from the culture supernatant obtained either from shake flask or fermentation cultivation.

First, cells and solids were removed from the fermentation culture medium by centrifugation for 10 min, 4000 g at 4°C. 10 mL of the culture supernatant was used for protein purification. The sample was filtered through 0.44 µm PVDF membrane (Millex-HV, Merck Millipore Ltd, Carrigtwohill, IRL) prior to loading into column.

Samples were loaded onto a HiPrep 26/10 Desalting column (GE Healthcare, Uppsala, Sweden) equilibrated in 25 mM Na-acetate pH 5. The desalted sample was then loaded onto a SP FF 5 ml column (GE Healthcare, Uppsala, Sweden) pre-equilibrated with 25 mM Na-acetate pH 5. After sample loading, the column was washed with the same buffer (30 ml). Proteins were eluted with linear salt gradient using 20 mM Na-acetate, 500 mM NaCl pH 5 in 15 CVs. Fractions of 5 ml were collected and analyzed on SDS-PAGE. The fractions containing target protein were combined and concentrated to 2 ml using Vivaspin 20, 10 kDa MWCO ultrafiltration devices (GE Healthcare). The concentrated sample was further fractionated using Superdex 75 26/60 gel-filtration column equilibrated with 20 mM Na-acetate, 150 mM NaCl pH 5. Fractions of 2 ml were collected and analyzed by SDS-PAGE. Fractions containing pure phytase were combined.

### Example 4. Phytase activity assays

Phytase activity was analysed using sodium phytate (C6H6Na12O24P6, LabChem EE05501) as a substrate in a concentration of 12.7 mM.

The activity of samples from the microtiter plate cultivations (Example 2) was screened using Fluent^{®} automation workstation (Tecan Group Ltd, Männedorf, Switzerland) as follows. The samples used in the assay are diluted in a 0.2 M citrate buffer (pH 5.0) containing 0.01 % Tween 20 (Merck 822184). 0.01 % of Tween 20 is added also to the substrate solution. 200 µl of sample dilution and 200 µl of substrate are mixed and incubated at 37 °C. After exactly 15 min incubation 400 µl of 15 % (w/v) TCA solution (Trichloroacetic acid, CCl3COOH, Merck 807) is added to the mixture to stop the reaction. 25 µl of reaction mixture is transferred into another well and 225 µl of water is added to make 1:10 dilution. 250 µl of colour reagent consisting of three volumes of 1 M sulphuric acid (H2SO4, Merck 731), one volume of 2.5 % (w/v) ammonium molybdate ((NH4)6Mo7O24 · 4 H2O, Merck 1182) and one volume of 10 % (w/v) ascorbic acid (C6H8O6, AnalaR Normapur 20150) is added and the colour reaction is incubated for 20 min at 50 °C. After the incubation the absorption is measured at 820 nm. The absorbance of the sample is compared to that of a parent sample, SEQ ID NO:1.

The activity from the shake flask and fermentation cultivations (Example 2) and from purified phytase preparations was analysed using a phytase activity assay (PPU). In PPU analysis one activity unit (PPU) is the quantity of enzyme that liberates 1 µmol of inorganic phosphate per one minute from sodium phytate at pH 5.0 and at 37 °C in a 15 min reaction time.

The samples used in the PPU assay are diluted in a reaction buffer (0.2 M citrate buffer, pH 5.0) and 1 ml of enzyme solution is used in the analysis. 1 ml of substrate is added to the enzyme solution and after incubating the mixture at 37 °C for exactly 15 min, the reaction is stopped by adding 2 ml of 15 % (w/v) TCA solution (Trichloroacetic acid, CCl3COOH, Merck 807). The reaction mixture is cooled to room temperature and after this 1:10 dilution is done by mixing 0.2 ml of the mixture and 1.8 ml of water in a test tube. 2.0 ml of freshly made colour reagent is added to the tube and mixed. The colour reagent consists of three volumes of 1 M sulphuric acid (H2SO4, Merck 731), one volume of 2.5 % (w/v) ammonium molybdate ((NH4)6Mo7O24 · 4 H2O, Merck 1182) and one volume of 10 % (w/v) ascorbic acid (C6H8O6, AnalaR Normapur 20150). The tubes are incubated at 50 °C for 20 min and cooled to room temperature. After this the absorption is measured at 820 nm against the enzyme blank. For the enzyme blank the substrate is added after the TCA and the 15 min incubation is passed. The amount of liberated phosphate is determined via a standard curve of the color reaction with a phosphate solution of known concentration.

The activity for the samples used in gastrointestinal tract test (GIT) (Example 7) was analysed by an internal validated phytase method (FTU assay). In FTU assay inorganic phosphate released from sodium phytate substrate by the hydrolytic enzymatic action of phytase is detected. Colour formation, which is measured spectrophotometrically, is the result of molybdate and vanadate ions complexing with inorganic phosphate. One phytase unit (FTU) is the quantity of enzyme that liberates 1 µmol of inorganic phosphate per minute from sodium phytate at 37 °C, pH 5.50, using 60 min incubation time.

In the assay, 2.0 ml of 1 % sodium phytate substrate (LabChem EE05501, in 250 mM sodium acetate buffer, pH 5.5 and including 1 mM CaCl2·2H2O and 0.01% Tween 20) is pipetted to plastic centrifuge tubes. The substrate tubes are pre-incubated for 5 -10 minutes at 37 °C after which 1.0 ml of diluted enzyme sample is added. After exactly 60 min incubation 2.0 ml of colour stop solution is added and tube contents are mixed by vortexing. Enzyme blanks are prepared like the sample but the colour stop solution is added to the substrate tubes prior to addition of the diluted enzyme sample. For colour reaction the tubes are incubated for 20 min at room temperature after which they are centrifuged at 4000 rpm for 10 minutes. The sample absorbance is measured against an enzyme blank at 415 nm. For the activity units, a potassium phosphate standard curve (pH 5.50) is prepared (dried KH2PO4, Merck 1.04873.1 is used for the standard; drying at 105 °C for 2 hours before weighting).

The stop solution is prepared as follows (preparation just prior to use): for 100 ml of colour stop solution, 25 ml of stock ammonium heptamolybdate (20 g of (NH4)6Mo7O24 · 4H2O, Merck 1182 in 180 ml of water, add 2 ml of ammonium hydroxide (NH4OH, Sigma-Aldrich 221228 28-30 %), final volume 200 ml) is mixed with 25 ml of stock ammonium vanadate solution (0.47 g of ammonium vanadate (NH4VO3, Riedel de Haen 31153) in 160 ml of water; once the completely dissolved, 4 ml of 22.75 % nitric acid solution is added, final volume 200 ml). Then, 16.5 ml of 22.75 % nitric acid solution (HNO3, Merck 1.00456) is added after which distilled water is added to make up the volume to 100 ml in volumetric flask.

The phytase activities of selected variants from culture supernatants as related to reference (SEQ ID NO:1) are shown in Table 2. Clearly improved relative phytase activities were obtained.

**Table 2. Phytase activities of selected variants. The numbers presented are relative activities compared to SEQ ID NO: 1.**

| **Variant code** | **Phytase activity** |
|---|---|
| SEQ ID NO: 1 | 1.00 |
| 00161 | 1.00 |
| 00173 | 1.15 |
| 00928 | 1.68 |
| 00933 | 1.4 |
| 00937 | 1.65 |
| BB10 | 1.22 |
| BB16 | 2.20 |
| BB19 | 1.90 |
| BB37 | 2.07 |
| BB38 | 1.63 |
| BB39 | 1.98 |
| BB40 | 1.47 |
| BB41 | 1.41 |
| BB42 | 1.66 |
| BB43 | 1.25 |
| BB44 | 1.37 |
| BB45 | 0.62 |
| BB46 | 1.59 |
| BB47 | 1.61 |
| BB48 | 1.54 |
| BB49 | 1.91 |
| BB50 | 1.60 |
| BB51 | 1.01 |
| BB52 | 1.34 |
| BB53 | 1.81 |
| BB54 | 2.31 |
| BB55 | 2.07 |
| BB56 | 2.38 |
| BB57 | 2.01 |
| BB58 | 2.07 |
| BB59 | 2.24 |
| BB60 | 2.04 |
| BB61 | 2.01 |
| BB62 | 2.20 |
| BB63 | 1.37 |

### Example 5. Analysis of unfolding temperatures of phytases

The unfolding temperatures of phytase variants (Examples 1 and 2) were analysed from culture supernatants using Prometheus NT.48 equipment (NanoTemper GmbH, Munich, Germany).

Prior to the measurements the samples were centrifuged for 10 min at 16000g at 4°C to remove any large aggregates. Standard-grade glass capillaries (NanoTemper GmbH) were filled with 10-15 µl of the sample, excitation light was preadjusted to get adequate fluorescence signal for F330 and F350, and samples were measured in temperature range 20―110°C with temperature slope of 1°C/min.

The inflection point of the unfolding curve, also known as the melting temperature (Tm), is determined from the experimental derivative or curve fitting. Protein samples with higher Tm tend to be more stable.

The parent phytase (SEQ ID NO:1) was used as a reference in the analysis.

The unfolding temperatures of the best variants were improved up to 3.6°C. The results from Prometheus analysis of selected variants are shown in Table 3.

**Table 3. Unfolding temperatures of parent and selected phytase variants. The unfolding temperatures were measured using Prometheus equipment NT.48.**

| **Variant code** | **Unfolding temperature (°C)** |
|---|---|
| SEQ ID NO: 1 | 88.7 |
| 00161 | 89.0 |
| 00173 | 89.7 |
| 00928 | 88.8 |
| 00933 | 89.4 |
| 00937 | 89.1 |
| BB10 | 89.4 |
| BB16 | 90.0 |
| BB19 | 90.9 |
| BB37 | 89.5 |
| BB38 | 89.7 |
| BB39 | 89.6 |
| BB40 | 91.1 |
| BB41 | 91.1 |
| BB42 | 92.3 |
| BB43 | 91.8 |
| BB44 | 91 |
| BB45 | 90.1 |
| BB46 | 90.1 |
| BB47 | 90.5 |
| BB48 | 90.3 |
| BB49 | 91.3 |
| BB50 | 92.1 |
| BB51 | 91.5 |
| BB52 | 89.3 |
| BB53 | 90.5 |
| BB54 | 88.1 |
| BB55 | 88.7 |
| BB56 | 88.5 |
| BB57 | 89.8 |
| BB58 | 90.6 |
| BB59 | 90.1 |
| BB60 | 90.2 |
| BB61 | 90.8 |
| BB62 | 90.1 |
| BB63 | 90.6 |

### Example 6. Stability test in feed processing

For this stability test in feed processing the current phytase sample was added to a wheat-soya bean meal based diet which was conditioned for 30 seconds at temperatures of 80°C and 95°C followed by pelleting through a 3 mm die. The pellets were cooled and analysed for phytase activity.

The feed produced contained approximately 65% wheat, 28% soybean meal, 4% soya oil, 1% monocalcium phosphate, 1.4% limestone, 0.4% salt and a premix (0.5%) of vitamins and trace minerals. Part of that feed was used to produce a premix with the phytase, that was added to the total feed amount and mixed in a horizontal mixer for 10 minutes. After taking the mash sample the feed was heated to the target temperature by adjusting steam addition when passing the cascade mixer before running though the pellet die. For each temperature, a sample was taken 10 minutes after the target conditioning temperature was achieved (as measured in the feed just prior to pelleting).

Samples of pelleted feeds were taken and cooled by cold air stream. On a sample splitter feeds were homogeneously sub-sampled for analysis using the phytase in feed assay method (ISO 30024:2009; Animal feeding stuffs ― Determination of phytase activity).

Phytase recovery was calculated relative to the activity in the corresponding feed mash sample pre-processing.

The phytase recovery compared to the corresponding activity in the mash feed was higher for phytase variant BB19 compared to phytase SEQ ID NO:1 after feed processing at 80°C and 95°C. This demonstrates that the improved unfolding temperature of BB19 translates into high stability under conditions common in commercial feed pelleting. The SEQ ID NO: 1 was used as a reference in the assay. The results are shown in Fig. 3.

### Example 7. Gastrointestinal tract test (GIT) results

The comparison of selected novel phytase candidates in their ability to degrade phytate in feed materials was done using an in vitro gastrointestinal simulation test system (GIT). Phytases to be tested were added with the same phytase protein content to a cornsoyabean meal-based trial feed following a three-step continuous in vitro test simulating the animal digestive conditions. The reactions were run at 40°C and at corresponding pHs and changes of pH as in the crop, gizzard and small intestine of broilers. Additionally, digestive proteases were added. To succeed in the GIT assay, the phytase needs to have a combination of beneficial biochemical properties. It needs to resist and act at different pHs at the temperature of the digestive tract while being resistant to proteases of the digestive tract. Details of the *in vitro* test system are described by Sommerfeld et al., 2017.

At the end of the in vitro test inositol phosphates are extracted and phytase removed from the supernatant before analysis of inositol phosphates (IP6 - IP3) using high performance liquid chromatography method (HPLC) according to Blaabjerg et al. 2010 .

The SEQ ID NO: 1 was used as reference in the GIT test.

The results are shown in Fig. 2. The degradation of phytate by all the tested phytase variants was at least as efficient or even better compared to SEQ ID NO:1 phytase.

### Example 8. Feeding trials

The thermophilic phytase variants described can be used in animal feeding, alone or in combination with other enzymes, to improve the availability of phytate bound phosphorus.

The efficiency in animal feeding of some of the recombinant phytase variants of the invention was confirmed in growth performance studies with broilers. Ultrafiltrate of the fermentation broth including the recombinant phytase was dried and defined activity levels were applied to the trial diets and the performance of the birds was compared to broilers fed the same diet composition without phytase.

Broiler trial 1: Day old male Ross 308 broilers were distributed to the different treatments (14 pens x 14 birds each). Phosphorus (P) and calcium Ca) content of the corn-soybean meal based diet that also included 10 % rape seed meal was adequate in a positive control diet (PC; starter and grower feed: P content 7.4 and 7.2 g/kg and Ca 9.5 and 9 g/kg, respectively) or reduced in the basal diet (BD; starter and grower feed: P content 5.14 and 4.8 g/kg and Ca 8.0 and 7.5 g/kg, respectively). Phytase SEQ ID NO: 1 or variants BB19 or BB16 were added to BD treatment feeds with a defined activity of 250 FTU/kg feed.

Both phytases improved the body weight (Fig 4 A) compared to birds fed the unsupplemented basal diet by 8%. Additionally, the increased body weight was achieved by a lower feed intake leading to an improvement of the feed conversion ratio (FCR; Fig 4 B) 6 and 7% by BB16 and BB19, respectively. The performance of the variants improved in thermostability was numerically better than for birds fed the same level of SEQ ID NO:1 phytase that improve body weight and FCR by 10 and 5%, respectively,

**Broiler trial 2**: Day old male Cobb 430 broilers were distributed to the different treatments (15 pens x 25 birds each). Phosphorus and calcium content of the corn-soybean meal diet was reduced in the basal diet (BD; starter feed: available P content 2.1 g/kg and Ca 8.5 g/kg, respectively). BB58 phytase were added to BD treatment feeds with defined activity levels of 125, 250 or 500 FTU/kg feed.

BB58 phytase improved the body weight (Fig 4 C) compared to birds fed the unsupplemented basal diet by 9, 16 and 23% in a clear dose response for 125, 250 and 500 FTU/kg, respectively. The FCR was also improved (Fig. 4 D).

**Broiler trial 3**: Day old male Ross 308 broilers were distributed to the different treatments (14 pens x 14 birds each). Phosphorus and calcium content of the corn-soybean meal diet that also included 8 % rape seed meal compared to the common recommendation was reduced in the basal diet (BD; P content 4.71 and Ca 8.5 g/kg). Phytase variant BB63 was added to BD treatment feeds with defined activities of 250 or 500 FTU/kg feed.

BB63 phytase improved the body weight (Fig 4 E) compared to birds fed the unsupplemented basal diet by 6 and 11% for 250 and 500 FTU/kg, respectively. Clear improvement in FCR was detected in birds fed using 500 FTU/kg dosing.

The results show that the variants with improved thermostability are efficient in animal feeding and the improvement in thermostability does not have negative effects on application performance. The results are shown in Fig. 4.

### REFERENCES

Ariza A, Moroz OV, Blagova EV, Turkenburg JP, Waterman J, Roberts SM, Vind J, Sjøholm C, Lassen SF, De Maria L, Glitsoe V, Skov LK and KS Wilson. 2013. Degradation of phytate by the 6-phytase from Hafnia alvei: a combined structural and solution study. PloS one 2013. 8(5), e65062. https://doi.org/10.1371/journal.pone.0065062
Bedford M.R. and C.L. Walk. 2016.Reduction of phytate to tetrakisphosphate (IP4) to trisphosphate (IP3), or perhaps even lower, does not remove its antinutritive properties. In: Phytate destruction - consequences for precision animal nutrition. Eds. Walk, C.L., Kühn, I., Stein, H.H., Kidd, M.T. and Rodehutscord, M. Wageningen 20 Academic publishers: 45-52.
Blaabjerg, K., H. Jørgensen, A. H. Tauson, and H. D. Poulsen. 2010. Heat-treatment, phytase and fermented liquid feeding affect the presence of inositol phosphates in ileal digesta and phosphorus digestibility in pigs fed a wheat and barley diet. Journal Article 4:876-885.
Greiner R. and U. Konietzny. 1996. Construction of a bioreactor to produce special breakdown products of phytate. Journal of Biotechnology, 48 (1-2): 153-159.
Havukainen S, Valkonen M, Koivuranta K and Landowski CP. 2020. Studies on sugar transporter CRT1 reveal new characteristics that are critical for cellulase induction in Trichoderma reesei. Biotechnol. Biofuels 2020 Sep 14;13:158. doi: 10.1186/s13068-020-01797-7. eCollection 2020.
Joutsjoki W, TK Torkkeli and KMH Nevalainen. 1993. Transformation of Trichoderma reesei with the Hormoconis resinae glucoamylase P (gamP) gene: production of a heterologous glucoamylase by Trichoderma reesei. Curr. Genet. 24:223-228.
Karhunen T, A Mäntylä, KMH Nevalainen and PL Suominen. 1993. High frequency one-step gene replacement in Trichoderma reesei. I. Endoglucanase I overproduction. Mol. Gen. Genet. 241:515-522.
Lee, S. A., and M. R. Bedford. 2016. Inositol- an effective growth promotor? Worlds Poultry Science 72, doi:10.1017/S0043933916000660
Menezes-Blackburn, D.,S. Gabler and R. Greiner. 2015. Performance of seven commercial phytases in an in Vitro simulation of poultry digestive tract. J. Agric. Food Chem., 63:6242-6149
Paloheimo M, A Mäntylä, J Kallio, and P Suominen. 2003. High-yield production of a bacterial xylanase in the filamentous fungus Trichoderma reesei requires a carrier polypeptide with an intact domain structure. Appl. Env. Microbiol. 69:7073-7082.
Penttilä M, H Nevalainen, M Rättö, E Salminen and J Knowles. 1987. A versatile transformation system for the cellulolytic filamentous fungus Trichoderma reesei. Gene 61:155-164.
Sambrook J and DW Russell. 2001. Molecular cloning, a laboratory manual. Cold Spring Harbor Laboratory, New York, US.
Sievers F, A Wilm, D Dineen, TJ Gibson, K Karplus, W Li, R Lopez, H McWilliam, M Remmert, J Söding, JD Thompson and DG Higgins 2011. Fast, scalable generation of high-quality protein multiple sequence alignments using Clustal Omega. Mol. Syst. Biol. 7:539.
Sommerfeld M, Schollenberger, L Hemberle and M Rodehutscord 2017 Modification and application of an in vitro assay to examine inositol phosphate degradation in the digestive tract of poultry. Science of Food and Agiculture; 97 (12), p 4219-4226; doi.org/10.1002/jsfa.8297
Xu P, J Price, A Wise and PJ Aggett. 1992. Interaction of Inositol Phosphates with Calcium, Zinc, and Histidine. Journal of Inorganic Biochemistry 47: 119 - 130.
Zeller E, M Schollenberger, I Kühn and M Rodehutscord. 2015. Hydrolysis of phytate and formation of inositol phosphate isomers without or with supplemented 30 phytases in different segments of the digestive tract of broilers. Journal Nutritional Science 4, e1: 1 - 12.

The foregoing description has provided by way of non-limiting examples of particular implementations and embodiments a full and informative description of the best mode presently contemplated by the inventors for carrying out the invention.

It is however clear to a person skilled in the art that the invention is not restricted to details of the embodiments presented in the foregoing, but that it can be implemented in other embodiments using equivalent means or in different combinations of embodiments without deviating from the characteristics of the invention.

## Claims

1. A variant polypeptide of an E. coli phytase comprising an amino acid sequence having at least 80% amino acid sequence identity with SEQ ID NO: 1, wherein the variant has:
a glutamic acid at the position 224;
phytase activity; and
wherein the amino acid numbering corresponds to the amino acid numbering of the SEQ ID NO: 1.

2. The variant polypeptide of claim 1 comprising at least one further amino acid substitution at a position selected from 30, 31, 35, 56, 67, 74, 80, 94, 107, 118, 120, 126, 140, 154, 174, 176, 177, 179, 180, 182, 183, 200, 202, 204, 211, 212, 227, 253, 271, 276, 277, 285, 287, 302, 315, 344, 352, 380, and 395.

3. The variant polypeptide of claim 1 or 2, wherein the E. *coli* phytase is a 6-phytase.

4. The variant polypeptide of any one of claims 1-3 having an increased thermostability, and/or an increased IP6 degrading activity, compared to a polypeptide having the amino acid sequence SEQ ID NO: 1.

5. The variant polypeptide of any one of claims 2-4, wherein the at least one further amino acid substitution results into the presence of at least one of the following amino acids: 30R/K, 31C, 35N, 56S, 67I/T, 74Q, 80P, 94L, 107N, 118L, 120R, 126H, 140C, 154N/E, 174E, 176P, 177C, 179K, 180N, 182K, 183A, 200C/I, 202S, 204N, 211V, 212G/A, 227E, 253Y/Q, 271I, 276M, 277A, 285E, 287S, 302A, 315G, 344D/E, 352M, 380P, and 395A, and wherein the amino acid numbering corresponds to the amino acid numbering of the SEQ ID NO: 1.

6. The variant polypeptide of any one of claims 2-5, wherein the at least one further amino acid substitution is a substitution selected from Q30R/K, D31C, D35N, A56S, V67I/T, K74Q, S80P, R94L, D107N, T118L, S120R, N126H, V140C, D154N/E, Q174E, N176P, L177C, L179K, K180N, E182K, K183A, V200C/I, A202S, D204N, W211V, S212G/A, Q227E, V253Y/Q, L271I, T277A, K276M, Q285E, Q287S, G302A, E315G, N344D/E, L352M, A380P, and G395A.

7. The variant polypeptide of any one of claims 1-6 comprising at least one additional amino acid substitution selected from 75C/V, 114T, 137E, 141T, 142D, 146R, 157G, 204C, 211W, 253Q, 267R, and 341P.

8. The variant polypeptide of any one of claims 1-7, wherein the variant polypeptide comprises:
a. the amino acids 30R, 74Q, 94L, 118L, 176P, 179K, 180N, 204N, 211V, 212G, 224E, 253Y, 315G, and 380P; or
b. the amino acids 30R, 94L, 118L, 176P, 179K, 180N, 204N, 211V, 212G, 224E, 253Y, 315G, 352M, and 380P; or
c. the amino acids 30R, 74Q, 94L, 118L, 176P, 179K, 180N, 204N, 211V, 212G, 224E, 253Y, 315G, 352M, and 380P; or
d. the amino acids 30R, 74Q, 80P, 94L, 118L, 176P, 179K, 180N, 204N, 211V, 212G, 224E, 227E, 253Y, 276M, 287S, 315G, 352M, and 380P; or
e. the amino acids 30R, 35N, 80P, 94L, 118L, 176P, 179K, 180N, 204N, 211V, 212G, 224E, 227E, 253Y, 276M, 287S, 315G, 352M, and 380P; or
f. the amino acids 30R, 80P, 94L, 118L, 176P, 179K, 180N, 183A, 204N, 211V, 212G, 224E, 227E, 253Y, 276M, 287S, 315G, 352M, and 380P; or
g. the amino acids 30R, 80P, 94L, 118L, 176P, 179K, 180N, 204N, 211V, 212G, 224E, 227E, 253Y, 276M, 287S, 315G, 352M, and 380P; or
h. the amino acids 30R, 74Q, 80P, 94L, 118L, 176P, 179K, 180N, 183A, 204N, 211V, 212G, 224E, 227E, 253Y, 276M, 287S, 315G, 352M, and 380P; or
i. the amino acids 30R, 94L, 118L, 126H, 176P, 179K, 180N, 204N, 211V, 212G, 224E, 253Y, 315G, 352M, and 380P; or
j. the amino acids 30R, 94L, 118L, 176P, 179K, 180N, 182K, 204N, 211V, 212G, 224E, 253Y, 315G, 352M, and 380P; or
k. the amino acids 30R, 74Q, 80P, 94L, 118L, 176P, 179K, 180N, 182K, 183A, 204N, 211V, 212G, 224E, 227E, 253Y, 276M, 287S, 315G, 352M, and 380P; or
I. the amino acids 30R, 35N, 74Q, 80P, 94L, 118L, 176P, 179K, 180N, 182K, 183A, 204N, 211V, 212G, 224E, 227E, 253Y, 276M, 287S, 315G, 352M, and 380P; or
m. the amino acids 30R, 35N, 74Q, 80P, 94L, 118L, 120R, 176P, 179K, 180N, 182K, 183A, 204N, 211V, 212G, 224E, 227E, 253Y, 276M, 287S, 315G, 352M, and 380P; or
n. the amino acids 30R, 31C, 74Q, 80P, 94L, 118L, 176P, 177C, 179K, 180N, 204N, 211V, 212G, 224E, 227E, 253Y, 276M, 287S, 315G, 352M, and 380P; or
o. the amino acids 30R, 35N, 74Q, 80P, 94L, 118L, 120R, 176P, 179K, 180N, 182K, 183A, 204N, 211V, 212G, 224E, 227E, 253Y, 276M, 287S, 315G, 352M, and 380P; or
p. the amino acids 30R, 80P, 94L, 118L, 140C, 176P, 179K, 180N, 200C, 204N, 211V, 212G, 224E, 227E, 253Y, 276M, 287S, 315G, 352M, and 380P; or
q. the amino acids 30R, 35N, 80P, 94L, 118L, 176P, 179K, 180N, 200I, 204N, 211V, 212G, 224E, 227E, 253Y, 276M, 287S, 315G, 352M, and 380P; or
r. the amino acids 30R, 56S, 67I, 74Q, 94L, 107N, 118L, 154N, 174E, 176P, 179K, 180N, 202S, 204N, 211V, 212G, 224E, 253Y, 2711, 285E, 302A, 315G, 344D, 380P, and 395A; or
s. the amino acids 30R, 56S, 67I, 94L, 107N, 118L, 154N, 174E, 176P, 179K, 180N, 202S, 204N, 211V, 212G, 224E, 253Y, 2711, 285E, 302A, 315G, 344D, 352M, 380P, and 395A; or
t. the amino acids 30R, 56S, 67I, 74Q, 94L, 107N, 118L, 154N, 174E, 176P, 179K, 180N, 202S, 204N, 211V, 212G, 224E, 253Y, 2711, 285E, 302A, 315G, 344D, 352M, 380P, and 395A; or
u. the amino acids 30R, 56S, 67I, 74Q, 80P, 94L, 107N, 118L, 154N, 174E, 176P, 179K, 180N, 202S, 204N, 211V, 212G, 224E, 227E, 253Y, 2711, 276M, 285E, 287S, 302A, 315G, 344D, 352M, 380P, and 395A; or
v. the amino acids 30R, 56S, 67I, 80P, 94L, 107N, 118L, 154N, 174E, 176P, 179K, 180N, 202S, 204N, 211V, 212G, 224E, 227E, 253Y, 2711, 276M, 285E, 287S, 302A, 315G, 344D, 352M, 380P, and 395A; or
w. the amino acids 30R, 35N, 56S, 67I, 74Q, 80P, 94L, 107N, 118L, 154N, 174E, 176P, 179K, 180N, 202S, 204N, 211V, 212G, 224E, 227E, 253Y, 2711, 276M, 285E, 287S, 302A, 315G, 344D, 352M, 380P, and 395A; or
x. the amino acids 30R, 35N, 56S, 67I, 80P, 94L, 107N, 118L, 154N, 174E, 176P, 179K, 180N, 202S, 204N, 211V, 212G, 224E, 227E, 253Y, 2711, 276M, 285E, 287S, 302A, 315G, 344D, 352M, 380P, and 395A; or
y. the amino acids 30R, 35N, 56S, 67I, 80P, 94L, 107N, 118L, 120R, 154N, 174E, 176P, 179K, 180N, 202S, 204N, 211V, 212G, 224E, 227E, 253Y, 2711, 276M, 285E, 287S, 302A, 315G, 344D, 352M, 380P, and 395A; or
z. the amino acids 30R, 56S, 67I, 80P, 94L, 107N, 118L, 154N, 176P, 179K, 180N, 202S, 204N, 211V, 212G, 224E, 227E, 253Y, 271I, 276M, 285E, 287S, 302A, 315G, 344D, 352M, 380P, and 395A; or
aa.the amino acids 30R, 56S, 671, 80P, 94L, 107N, 118L, 126H, 154N, 174E, 176P, 179K, 180N, 202S, 204N, 211V, 212G, 224E, 227E, 253Y, 2711, 276M, 285E, 287S, 302A, 315G, 344D, 352M, 380P, and 395A; or
bb.the amino acids 30R, 80P, 94L, 118L, 176P, 179K, 180N, 204N, 211V, 212G, 224E, 227E, 253Y, 276M, 277A, 287S, 315G, and 380P; or
cc. a set of amino acid substitutions specified in Table 1.

9. The variant polypeptide of any one of claims 1-8, wherein the variant polypeptide comprises the substitutions selected from:
a. Q30R, K74Q, R94L, T118L, N176P, L179K, K180N, D204N, W211V, S212G, Q224E, V253Y, E315G, and A380P; or
b. Q30R, R94L, T118L, N176P, L179K, K180N, D204N, W211V, S212G, Q224E, V253Y, E315G, L352M, and A380P; or
c. Q30R, K74Q, R94L, T118L, N176P, L179K, K180N, D204N, W211V, S212G, Q224E, V253Y, E315G, L352M, and A380P; or
d. Q30R, K74Q, S80P, R94L, T118L, N176P, L179K, K180N, D204N, W211V, S212G, Q224E, Q227E, V253Y, K276M, Q287S, E315G, L352M, and A380P; or
e. Q30R, D35N, S80P, R94L, T118L, N176P, L179K, K180N, D204N, W211V, S212G, Q224E, Q227E, V253Y, K276M, Q287S, E315G, L352M, and A380P; or
f. Q30R, S80P, R94L, T118L, N176P, L179K, K180N, K183A, D204N, W211V, S212G, Q224E, Q227E, V253Y, K276M, Q287S, E315G, L352M, and A380P; or
g. Q30R, S80P, R94L, T118L, N176P, L179K, K180N, D204N, W211V, S212G, Q224E, Q227E, V253Y, K276M, Q287S, E315G, L352M, and A380P; or
h. Q30R, K74Q, S80P, R94L, T118L, N176P, L179K, K180N, K183A, D204N, W211V, S212G, Q224E, Q227E, V253Y, K276M, Q287S, E315G, L352M, and A380P; or
i. Q30R, R94L, T118L, N126H, N176P, L179K, K180N, D204N, W211V, S212G, Q224E, V253Y, E315G, L352M, and A380P; or
j. Q30R, R94L, T118L, N176P, L179K, K180N, E182K, D204N, W211V, S212G, Q224E, V253Y, E315G, L352M, and A380P; or
k. Q30R, K74Q, S80P, R94L, T118L, N176P, L179K, K180N, E182K, K183A, D204N, W211V, S212G, Q224E, Q227E, V253Y, K276M, Q287S, E315G, L352M, and A380P; or
I. Q30R, D35N, K74Q, S80P, R94L, T118L, N176P, L179K, K180N, E182K, K183A, D204N, W211V, S212G, Q224E, Q227E, V253Y, K276M, Q287S, E315G, L352M, and A380P; or
m. Q30R, D35N, K74Q, S80P, R94L, T118L, S120R, N176P, L179K, K180N, E182K, K183A, D204N, W211V, S212G, Q224E, Q227E, V253Y, K276M, Q287S, E315G, L352M, and A380P; or
n. Q30R, D31C, K74Q, S80P, R94L, T118L, N176P, L177C, L179K, K180N, D204N, W211V, S212G, Q224E, Q227E, V253Y, K276M, Q287S, E315G, L352M, and A380P; or
o. Q30R, D35N, K74Q, S80P, R94L, T118L, S120R, N176P, L179K, K180N, E182K, K183A, D204N, W211V, S212G, Q224E, Q227E, V253Y, K276M, Q287S, E315G, L352M, and A380P; or
p. Q30R, S80P, R94L, T118L, V140C, N176P, L179K, K180N, V200C, D204N, W211V, S212G, Q224E, Q227E, V253Y, K276M, Q287S, E315G, L352M, and A380P; or
q. Q30R, D35N, S80P, R94L, T118L, N176P, L179K, K180N, V200I, D204N, W211V, S212G, Q224E, Q227E, V253Y, K276M, Q287S, E315G, L352M, and A380P; or
r. Q30R, A56S, V67I, K74Q, R94L, D107N, T118L, D154N, Q174E, N176P, L179K, K180N, A202S, D204N, W211V, S212G, Q224E, V253Y, L271I, Q285E, G302A, E315G, N344D, A380P, and G395A; or
s. Q30R, A56S, V67I, R94L, D107N, T118L, D154N, Q174E, N176P, L179K, K180N, A202S, D204N, W211V, S212G, Q224E, V253Y, L271I, Q285E, G302A, E315G, N344D, L352M, A380P, and G395A; or
t. Q30R, A56S, V67I, K74Q, R94L, D107N, T118L, D154N, Q174E, N176P, L179K, K180N, A202S, D204N, W211V, S212G, Q224E, V253Y, L271I, Q285E, G302A, E315G, N344D, L352M, A380P, and G395A; or
u. Q30R, A56S, V67I, K74Q, S80P, R94L, D107N, T118L, D154N, Q174E, N176P, L179K, K180N, A202S, D204N, W211V, S212G, Q224E, Q227E, V253Y, L271I, K276M, Q285E, Q287S, G302A, E315G, N344D, L352M, A380P, and G395A; or
v. Q30R, A56S, V67I, S80P, R94L, D107N, T118L, D154N, Q174E, N176P, L179K, K180N, A202S, D204N, W211V, S212G, Q224E, Q227E, V253Y, L271I, K276M, Q285E, Q287S, G302A, E315G, N344D, L352M, A380P, and G395A; or
w. Q30R, D35N, A56S, V67I, K74Q, S80P, R94L, D107N, T118L, D154N, Q174E, N176P, L179K, K180N, A202S, D204N, W211V, S212G, Q224E, Q227E, V253Y, L271I, K276M, Q285E, Q287S, G302A, E315G, N344D, L352M, A380P, and G395A; or
x. Q30R, D35N, A56S, V67I, S80P, R94L, D107N, T118L, D154N, Q174E, N176P, L179K, K180N, A202S, D204N, W211V, S212G, Q224E, Q227E, V253Y, L271I, K276M, Q285E, Q287S, G302A, E315G, N344D, L352M, A380P, and G395A; or
y. Q30R, D35N, A56S, V67I, S80P, R94L, D107N, T118L, S120R, D154N, Q174E, N176P, L179K, K180N, A202S, D204N, W211V, S212G, Q224E, Q227E, V253Y, L271I, K276M, Q285E, Q287S, G302A, E315G, N344D, L352M, A380P, and G395A; or
z. Q30R, A56S, V67I, S80P, R94L, D107N, T118L, D154N, N176P, L179K, K180N, A202S, D204N, W211V, S212G, Q224E, Q227E, V253Y, L271I, K276M, Q285E, Q287S, G302A, E315G, N344D, L352M, A380P, and G395A; or
aa.Q30R, A56S, V67I, S80P, R94L, D107N, T118L, N126H, D154N, Q174E, N176P, L179K, K180N, A202S, D204N, W211V, S212G, Q224E, Q227E, V253Y, L271I, K276M, Q285E, Q287S, G302A, E315G, N344D, L352M, A380P, and G395A;
bb. R94L and T118L/I; or
cc. Q174E, N176P, L179K, and K180N; or
dd.A202S and D204N; or
ee.A202S, D204N, S212G/A, and V253Y/Q; or
ff. L2711 and G302A; or
gg. Q285E and Q287S/K; or
hh. E315G and A380P; or
ii. Q30R, S80P, R94L, T118L, N176P, L179K, K180N, D204N, W211V, S212G, Q224E, Q227E, V253Y, K276M, T277A, Q287S, E315G, and A380P.

10. The variant polypeptide of any one of claims 1-9 having an increased relative IP6 degrading activity compared to the SEQ ID NO: 1 when expressed in an expression host.

11. The variant polypeptide of any one of claims 1-10 having both an increased thermostability and an increased relative IP6 degrading activity compared to the SEQ ID NO: 1.

12. A recombinant host cell comprising genetic elements configured to produce at least one variant polypeptide of any one of claims 1-11, wherein the host cell is preferably selected from the group consisting of
a. filamentous fungal cells from Division Ascomycota, Subdivision Pezizomycotina; preferably from the group consisting of members of the Class Sordariomycetes or Eurotiomycetes, Subclass Hypocreomycetidae or Sordariomycetidae or Eurotiomycetidae, Orders Hypocreales or Sordariales or Eurotiales, Families Hypocreacea or Nectriacea or Chaetomiaceae or Aspergillaceae, Genera *Trichoderma* (anamorph of *Hypocrea*) or *Fusarium* or *Acremonium* or *Humicola* or *Thermothelomyces* or *Myceliophthora* or *Aspergillus;*
more preferably from the group consisting of species *Trichoderma reesei* (*Hypocrea jecorina), T. citrinoviridae, T. longibrachiatum, T. virens, T. harzianum, T. asperellum, T. atroviridae, T. parareesei, Fusarium oxysporum, F. gramineanum, F. pseudograminearum, F. venenatum, Acremonium (Cephalosporium) chrysogenum,* , *Humicola insolens, H. grisea, Thermothelomyces thermophilus, Myceliophthora thermophila, Aspergillus niger, A. niger* var. *awamori* and *A. oryzae;*
b. bacterial cells, preferably gram-positive Bacilli such as *B*. *subtilis, B*. *licheniformis, B*. *megaterium, B*. *amyloliquefaciens, B*. *pumilus,* gram negative bacteria such as *Escherichia coli*, actinomycetales such as *Streptomyces* sp.; and
c. yeasts, such as *Saccharomyces cerevisiae, Schizosaccharomyces pombe, Pichia pastoris, Yarrowia lipolytica*; and
more preferably the host cell is selected from filamentous fungal cells such as *Trichoderma,* or from gram-positive Bacilli such as *Bacillus*;
most preferably from *Trichoderma reesei* or from *Bacillus subtilis* or *B*. *pumilus or B*. *licheniformis* or *B*. *amyloliquefaciens.*

13. A recombinant host cell comprising genetic elements configured to produce at least one variant polypeptide of any one of claims 1-11, and wherein the host cell is a transgenic plant cell.

14. An enzyme composition comprising the variant polypeptide of any one of claims 1-11.

15. A use of the variant polypeptide of any one of claims 1-11 or the enzyme composition of claim 14 in the manufacturing of feedstuff, foodstuff, feed additive, dietary supplement, or a pharmaceutical.

16. A method of manufacturing the variant polypeptide of any one of claims 1-11 comprising:
a. providing a polynucleotide comprising genetic elements arranged to produce the variant polypeptide of claims 1-11; and
b. expressing the polynucleotide in a recombinant host cell, preferably in the recombinant host cell of claim 12 or 13.

17. An animal feed comprising the variant polypeptide of any one of claims 1-11, or the enzyme composition of claim 14, and at least one protein source of plant origin, and
a. optionally at least one further enzyme selected from protease, amylase, phytase, xylanase, endoglucanase, beta-glucanase, mannanase, cellulase, or a combination thereof; and
b. optionally at least one filler selected from maltodextrin, flour, salt, sodium chloride, sulphate, sodium sulphate, or a combination thereof.

18. A feed supplement comprising the variant polypeptide of any one of claims 1-11 or the enzyme composition of claim 14; and
a. optionally at least one further enzyme selected from protease, amylase, phytase, xylanase, endoglucanase, beta-glucanase, mannanase, or a combination thereof; and
b. optionally at least one carrier or ingredient selected from maltodextrin, flour, salt, sodium chloride, sulphate, sodium sulphate, minerals, amino acids, prebiotics, probiotics, vitamins, or a combination thereof.

19. A method of degrading or modifying material containing phytic acid or phytate, comprising treating said material with an effective amount of the variant polypeptide of any one of claims 1-11 or the enzyme composition of claim 14.
